# EUROPEAN PATENT APPLICATION

(11) **EP 1 375 672 A1**
(43) Date of publication of application: **02.01.2004**
(21) Application number: 02702736.6
(22) Date of filing: 05.03.2002
(51) Int. Cl.: C12Q 1/68, C12N 15/09, C12M 1/00, G01N 33/53, G01N 33/543, G01N 33/566, G01N 33/576, G01N 37/00

(54) **METHOD OF DETECTING NUCLEIC ACID RELATING TO DISEASE**

(30) Priority: 27.03.2001 JP 2001090053; 18.09.2001 JP 2001284112
(71) Applicant: Kabushiki Kaisha Toshiba, Tokyo 105-8001 (JP)
(72) Inventor: HASHIMOTO, Koji, Sagamihara-shi, Kanagawa 229-0031 (JP); HASHIMOTO, Michie, Shibuya-ku, Tokyo 150-0001 (JP); MISHIRO, Shunji, Bunkyo-ku, Tokyo 113-0021 (JP); OOTA, Yasuhiko, Nakano-ku, Tokyo 165-0026 (JP)
(74) Representative: Andrews, Timothy Stephen
(86) International application number: PCT/JP2002/002030
(87) International publication number: WO 2002/077281

(57) **Abstract**

The present invention provides methods for obtaining information regarding nucleic acid from an individual and nucleic acid associated with a disease of the individual, in particular when the disease is associated with a pathogenic microorganism present within the individual. The present invention also provide probe-immobilized substrates, such as probe-immobilized chips, for use in the methods. In particular, the present invention provides methods and probe-immobilized substrates for obtaining information regarding responsiveness to a treatment for a disease of an individual.

SEQUENCE LISTING

<110>  KABUSHIKI KAISHA TOSHIBA
       HASHIMOTO, Koji
       HASHIMOTO, Michie
       MISHIRO, Shunji
       OOTA, Yasuhiko

<120>  DETECTION OF NUCLEIC ACID ASSOCIATED WITH DISEASE

<130>  01S1691P

<150>  JP 2001-90053
<151>  2001-3-27
<150>  JP 2001-284112
<151>  2001-9-18

<160> 72

<210> 1
<211> 21
<212> DNA
<213> Hepatitis C Virus
<400> 1
ccctgtgagg aactwctgtc t                                                21

<210> 2
<211> 21
<212> DNA

<213> Hepatitis C Virus

<400> 2

ggtgcacggt ctacgagacc t                                                21

<210> 3

<211> 26

<212> DNA

<213> Hepatitis C Virus

<400> 3

tctagccatg gcgttagtry gagtgt                                           26

<210> 4

<211> 26

<212> DNA

<213> Hepatitis C Virus

<400> 4

cactcgcaag caccctatca ggcagt                                           26

<210> 5

<211> 18

<212> DNA

<213> Hepatitis C Virus

<400> 5

cgctcaatgc ctggagat                                                    18

<210> 6

<211> 18

<212> DNA

<213> Hepatitis C Virus

<400> 6
cactctatgc ccggccat                                                    18

<210> 7
<211> 18
<212> PNA
<213> Hepatitis C Virus
<400> 7
cgctcaatac ccagaaat                                                    18

<210> 8
<211> 20
<212> DNA
<213> Hepatitis C Virus
<400> 8
cgcgcgacta ggaagacttc                                                  20

<210> 9
<211> 20
<212> DNA
<213> Hepatitis C Virus
<400> 9
cgcgcgacgc gcaaaacttc                                                  20

<210> 10
<211> 20
<212> DNA
<213> Hepatitis C Virus
<400> 10

tgccttgggg ataggctgac 20

<210> 11

<211> 20

<212> DNA

<213> Hepatitis C Virus

<400> 11

gagccatcct gcccacccca 20

<210> 12

<211> 20

<212> DNA

<213> Hepatitis C Virus

<400> 12

gccccatgaa gggcgagaac 20

<210> 13

<211> 20

<212> DNA

<213> Hepatitis C Virus

<400> 13

accctcgttt ccgtacagag 20

<210> 14

<211> 20

<212> DNA

<213> Hepatitis C Virus

<400> 14

gctgagccca ggaccggtct 20

<210> 15

<211> 20

<212> DNA

<213> Hepatitis C Virus

<400> 15

aggaagactt ccgagcggtc                                                20

<210> 16

<211> 581

<212> DNA

<213> Homo sapiens

<400> 16

atgagccaga ctccagggag gcctagaagt gggcaagggg aaacgggaaa ggaggaagat 60

ggtatgggtg tgcctggtta ggggtgggag tgctggacgg agttcgggac aagaggggct 120

ctgcagccat tggcacacaa tgcctgggag tccctgctgg tgctgggatc atcccagtga 180

gccctgggag ggaactgaag acccccaatt accaatgcat ctgttttcaa aaccgacggg 240

gggaaggaca tgcctaggtt caaggatacg tgcaggcttg gatgactccg ggccattagg 300

gagcctccgg agcaccttga tcctcagacg ggcctgatga aacgagcatc tgattcagca 360

ggcctgggtt cgggcccgag aacctgcgtc tcccgcgagt tcccgcgagg caagtgctgn 420

aggtgcgggg ccaggagcta ggtttcgttt ctgctcccgg agccgccctc agcacagggt 480

ctgtgagttt catttcttcg ccggcgcggg gcggggctgg gcgcggggtg aaagaggcga 540

accgagagcg gaggccgcac tccagcactg cgcagggacc g                        581

<210> 17

<211> 581

<212> DNA

<213> Homo sapiens

<400> 17

atgagccaga ctccagggag gcctagaagt gggcaagggg aaacgggaaa ggaggaagat 60
ggtatgggtg tgcctggtta ggggtgggag tgctggacgg agttcgggac aagaggggct 120
ctgcagccat tggcacacaa tgcctgggag tccctgctgg tgctgggatc atcccagtga 180
gccctgggag ggaactgaag acccccaatt accaatgcat ctgttttcaa aaccgacggg 240
gggaaggaca tgcctaggtt caaggatacg tgcaggcttg gatgactccg ggccattagg 300
gagcctccgg agcaccttga tcctcagacg ggcctgatga aacgagcatc tgattcagca 360
ggcctgggtt cgggcccgag aacctgcgtc tcccgcgagt tcccgcgagg caagtgctgn 420
aggtgcgggg ccaggagcta ggtttcgttt ctgcgcccgg agccgccctc agcacagggt 480
ctgtgagttt catttcttcg ccggcgcggg gcggggctgg gcgcggggtg aaagaggcga 540
accgagagcg gaggccgcac tccagcactg cgcagggacc g 581

<210> 18
<211> 581
<212> DNA
<213> Homo sapiens
<400> 18
atgagccaga ctccagggag gcctagaagt gggcaagggg aaacgggaaa ggaggaagat 60
ggtatgggtg tgcctggtta ggggtgggag tgctggacgg agttcgggac aagaggggct 120
ctgcagccat tggcacacaa tgcctgggag tccctgctgg tgctgggatc atcccagtga 180
gccctgggag ggaactgaag acccccaatt accaatgcat ctgttttcaa aaccgacggg 240
gggaaggaca tgcctaggtt caaggatacg tgcaggcttg gatgactccg ggccattagg 300
gagcctccgg agcaccttga tcctcagacg ggcctgatga aacgagcatc tgattcagca 360
ggcctgggtt cgggcccgag aacctgcgtc tcccgcgagt tcccgcgagg caagtgctgn 420
aggtgcgggg ccaggagcta ggtttcgttt ctgcacccgg agccgccctc agcacagggt 480
ctgtgagttt catttcttcg ccggcgcggg gcggggctgg gcgcggggtg aaagaggcga 540
accgagagcg gaggccgcac tccagcactg cgcagggacc g 581

<210> 19
<211> 581

<212> DNA

<213> Homo sapiens

<400> 19

atgagccaga ctccagggag gcctagaagt gggcaagggg aaacgggaaa ggaggaagat 60

ggtatgggtg tgcctggtta ggggtgggag tgctggacgg agttcgggac aagaggggct 120

ctgcagccat tggcacacaa tgcctgggag tccctgctgg tgctgggatc atcccagtga 180

gccctgggag ggaactgaag acccccaatt accaatgcat ctgttttcaa aaccgacggg 240

gggaaggaca tgcctaggtt caaggatacg tgcaggcttg gatgactccg ggccattagg 300

gagcctccgg agcaccttga tcctcagacg ggcctgatga aacgagcatc tgattcagca 360

ggcctgggtt cgggcccgag aacctgcgtc tcccgcgagt tcccgcgagg caagtgctgn 420

aggtgcgggg ccaggagcta ggtttcgttt ctgcccccgg agccgccctc agcacagggt 480

ctgtgagttt catttcttcg ccggcgcggg gcggggctgg gcgcggggtg aaagaggcga 540

accgagagcg gaggccgcac tccagcactg cgcagggacc g 581

<210> 20

<211> 22

<212> DNA

<213> Homo sapiens

<400> 20

aggtgcgggg ccaggagcta gg 22

<210> 21

<211> 22

<212> DNA

<213> Homo sapiens

<400> 21

ggcctccgct ctcgcttcgc ct 22

<210> 22

<211> 19

<212> DNA

<213> Homo sapiens

<400> 22

tcgtttctgc tcccggagc 19

<210> 23

<211> 19

<212> DNA

<213> Homo sapiens

<400> 23

tcgtttctgc gcccggagc 19

<210> 24

<211> 19

<212> DNA

<213> Homo sapiens

<400> 24

tcgtttctgc ccccggagc 19

<210> 25

<211> 19

<212> DNA

<213> Homo sapiens

<400> 25

tcgtttctgc acccggagc 19

<210> 26

<211> 20

<212> DNA
<213> Homo sapiens
<400> 26
cttgtctcgt agctgcagcc                                                    20

<210> 27
<211> 15
<212> PNA
<213> Homo sapiens
<400> 27
gtttctgctc ccgga                                                    15

<210> 28
<211> 15
<212> PNA
<213> Homo sapiens
<400> 28
gtttctgcgc ccgga                                                    15

<210> 29
<211> 15
<212> PNA
<213> Homo sapiens
<400> 29
gtttctgccc ccgga                                                    15

<210> 30
<211> 15
<212> PNA

<213> Homo sapiens

<400> 30

gtttctgcac ccgga                                                    15

<210> 31

<211> 15

<212> PNA

<213> Homo sapiens

<400> 31

tgctgtcgat cgcac                                                    15

<210> 32

<211> 15

<212> PNA

<213> Hepatitis C Virus

<400> 32

cttggggata ggctg                                                    15

<210> 33

<211> 15

<212> PNA

<213> Hepatitis C Virus

<400> 33

ccatcctgcc caccc                                                    15

<210> 34

<211> 15

<212> PNA

<213> Hepatitis C Virus

<400> 34
ccatgaaggg cgaga                                                    15

<210> 35
<211> 15
<212> PNA
<213> Hepatitis C Virus
<400> 35
ctcgtttccg tacag                                                    15

<210> 36
<211> 15
<212> PNA
<213> Hepatitis C Virus
<400> 36
gagcccagga ccggt                                                    15

<210> 37
<211> 581
<212> DNA
<213> Homo sapiens
<400> 37
atgagccaga ctccagggag gcctagaagt gggcaagggg aaacgggaaa ggaggaagat 60
ggtatgggtg tgcctggtta ggggtgggag tgctggacgg agttcgggac aagaggggct 120
ctgcagccat tggcacacaa tgcctgggag tccctgctgg tgctgggatc atcccagtga 180
gccctgggag ggaactgaag acccccaatt accaatgcat ctgttttcaa aaccgacggg 240
gggaaggaca tgcctaggtt caaggatacg tgcaggcttg gatgactccg ggccattagg 300
gagcctccgg agcaccttga tcctcagacg ggcctgatga aacgagcatc tgattcagca 360

ggcctgggtt cgggcccgag aacctgcgtc tcccgcgagt tcccgcgagg caagtgctgt 420
aggtgcgggg ccaggagcta ggtttcgttt ctgcncccgg agccgccctc agcacagggt 480
ctgtgagttt catttcttcg ccggcgcggg gcggggctgg gcgcggggtg aaagaggcga 540
accgagagcg gaggccgcac tccagcactg cgcagggacc g 581

<210> 38
<211> 581
<212> DNA
<213> Homo sapiens
<400> 38
atgagccaga ctccagggag gcctagaagt gggcaagggg aaacgggaaa ggaggaagat 60
ggtatgggtg tgcctggtta ggggtgggag tgctggacgg agttcgggac aagaggggct 120
ctgcagccat tggcacacaa tgcctgggag tccctgctgg tgctgggatc atcccagtga 180
gccctgggag ggaactgaag acccccaatt accaatgcat ctgttttcaa aaccgacggg 240
gggaaggaca tgcctaggtt caaggatacg tgcaggcttg gatgactccg ggccattagg 300
gagcctccgg agcaccttga tcctcagacg ggcctgatga aacgagcatc tgattcagca 360
ggcctgggtt cgggcccgag aacctgcgtc tcccgcgagt tcccgcgagg caagtgctgg 420
aggtgcgggg ccaggagcta ggtttcgttt ctgcncccgg agccgccctc agcacagggt 480
ctgtgagttt catttcttcg ccggcgcggg gcggggctgg gcgcggggtg aaagaggcga 540
accgagagcg gaggccgcac tccagcactg cgcagggacc g 581

<210> 39
<211> 581
<212> DNA
<213> Homo sapiens
<400> 39
atgagccaga ctccagggag gcctagaagt gggcaagggg aaacgggaaa ggaggaagat 60

ggtatgggtg tgcctggtta ggggtgggag tgctggacgg agttcgggac aagaggggct 120
ctgcagccat tggcacacaa tgcctgggag tccctgctgg tgctgggatc atcccagtga 180
gccctgggag ggaactgaag acccccaatt accaatgcat ctgttttcaa aaccgacggg 240
gggaaggaca tgcctaggtt caaggatacg tgcaggcttg gatgactccg ggccattagg 300
gagcctccgg agcaccttga tcctcagacg ggcctgatga aacgagcatc tgattcagca 360
ggcctgggtt cgggcccgag aacctgcgtc tcccgcgagt tcccgcgagg caagtgctga 420
aggtgcgggg ccaggagcta ggtttcgttt ctgcncccgg agccgccctc agcacagggt 480
ctgtgagttt catttcttcg ccggcgcggg gcggggctgg gcgcggggtg aaagaggcga 540
accgagagcg gaggccgcac tccagcactg cgcagggacc g 581

<210> 40
<211> 581
<212> DNA
<213> Homo sapiens
<400> 39
atgagccaga ctccagggag gcctagaagt gggcaagggg aaacgggaaa ggaggaagat 60
ggtatgggtg tgcctggtta ggggtgggag tgctggacgg agttcgggac aagaggggct 120
ctgcagccat tggcacacaa tgcctgggag tccctgctgg tgctgggatc atcccagtga 180
gccctgggag ggaactgaag acccccaatt accaatgcat ctgttttcaa aaccgacggg 240
gggaaggaca tgcctaggtt caaggatacg tgcaggcttg gatgactccg ggccattagg 300
gagcctccgg agcaccttga tcctcagacg ggcctgatga aacgagcatc tgattcagca 360
ggcctgggtt cgggcccgag aacctgcgtc tcccgcgagt tcccgcgagg caagtgctgc 420
aggtgcgggg ccaggagcta ggtttcgttt ctgcncccgg agccgccctc agcacagggt 480
ctgtgagttt catttcttcg ccggcgcggg gcggggctgg gcgcggggtg aaagaggcga 540
accgagagcg gaggccgcac tccagcactg cgcagggacc g 581

<210> 41

<211> 1802

<212> DNA

<213> Homo sapiens

<400> 41

gaattcctgc cagaaagtag agaggtattt agcactctgc cagggccaac gtagtaagaa 60

atttccagag aaaatgctta cccaggcaag cctgtntaaa acaccaaggg gaagcaaact 120

ccagttaatt ctgggctggg ttggtgacta aggttgaggt tgatctgagg ttgagacctt 180

cctctttgga tcaccagctt tcagctcagg gcctgccaat gagtaaatga tagttaacag 240

gtcctggagg ggaatcagct gcccagatac aaagatggga ttcaggtggc agatggaccc 300

gaagaggaca tggagagaaa gaggaagctc ctacagacac ctgggtttcc actcattctc 360

attccctaag ctaacaggca taagccagct ggcaatgcac ggtcccattt gttctcactg 420

ccacggaaag catgtttata gtcttccagc agcaacgcca ggtgtctagg cacagatgaa 480

cccctcctta ggatccccac tgctcatcat agtgcctacc tttgttaaag tactagtcac 540

gcagtgtcac aaggaatgtt tacttttcca aatccccagc tagaggccag ggatgggtca 600

tctatttcta tatagcctgc acccagattg taggacagag ggcatgctng gtaaatatgt 660

gttcattaac tgagattaac cttccctgag ttttctcaca ccaaggtgag gaccatgtcc 720

ctgtttccat cactccctct ccttctcctg agtatggtgg cagcgtctta ctcagaaact 780

gtgacctgtg aggatgccca aaagacctgc cctgcagtga ttgcctgtag ctctccaggc 840

atcaacggct tcccaggcaa agatgggngt gatgncacca aggnagaaaa gggggaacca 900

ggtacgtgtt gggctgttct gtctctgcaa ttctttacct tccagaggaa actgcctggg 960

gatatgagga gactgatgtc ctatttgagt atatttttct caactatact gtaactcaaa 1020

acagagattc agctcgaatt ccacacagca gtttgtgact aatagttgtc ttgccagccc 1080

aggaaagtgg cccacaggtc aggccatccc gtgggacaca ggatgaattt ttcttctctg 1140

ggtcattgtc atgtcagacc cctattcact tcagtaggga tggcaccagg ttcaagaggc 1200

caaagaagag atggagtcag caaacaaaca taggttttac tgggggaatc tgtttacagg 1260

gagatccagc agcagtgggc tggacaggag aacaacaact actggtaaaa acaaatgcag 1320

ttaattttca ctttgcaccc tccctgcagc aacctccacg tggcaacttt atttcttaag 1380

ttattgctct caggtgcaca ccatacagtt attgagagca gtgctcagaa aggtcagtcc 1440

tgggtcaagg tctcccttct cctgagaagg gattgggcat caaactcttg aagagagaga 1500

gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 42
<211> 1802
<212> DNA
<213> Homo sapiens

<400> 42
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccaccgaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatgncacca aggnagaaaa gggggaacca 900
ggtacgtgtt gggctgttct gtctctgcaa ttctttacct tccagaggaa actgcctggg 960

gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 43
<211> 1802
<212> DNA
<213> Homo sapiens

<400> 43
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420

ccacagaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatgncacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 44
<211> 1802
<212> DNA
<213> Homo sapiens

<400> 44

gaattcctgc cagaaagtag agaggtattt agcactctgc cagggccaac gtagtaagaa 60

atttccagag aaaatgctta cccaggcaag cctgtntaaa acaccaaggg gaagcaaact 120

ccagttaatt ctgggctggg ttggtgacta aggttgaggt tgatctgagg ttgagacctt 180

cctctttgga tcaccagctt tcagctcagg gcctgccaat gagtaaatga tagttaacag 240

gtcctggagg ggaatcagct gcccagatac aaagatggga ttcaggtggc agatggaccc 300

gaagaggaca tggagagaaa gaggaagctc ctacagacac ctgggtttcc actcattctc 360

attccctaag ctaacaggca taagccagct ggcaatgcac ggtcccattt gttctcactg 420

ccactgaaag catgtttata gtcttccagc agcaacgcca ggtgtctagg cacagatgaa 480

cccctcctta ggatccccac tgctcatcat agtgcctacc tttgttaaag tactagtcac 540

gcagtgtcac aaggaatgtt tacttttcca aatccccagc tagaggccag ggatgggtca 600

tctatttcta tatagcctgc acccagattg taggacagag ggcatgctng gtaaatatgt 660

gttcattaac tgagattaac cttccctgag ttttctcaca ccaaggtgag gaccatgtcc 720

ctgtttccat cactccctct ccttctcctg agtatggtgg cagcgtctta ctcagaaact 780

gtgacctgtg aggatgccca aaagacctgc cctgcagtga ttgcctgtag ctctccaggc 840

atcaacggct tcccaggcaa agatgggngt gatgncacca aggnagaaaa gggggaacca 900

ggtacgtgtt gggctgttct gtctctgcaa ttctttacct tccagaggaa actgcctggg 960

gatatgagga gactgatgtc ctatttgagt atatttttct caactatact gtaactcaaa 1020

acagagattc agctcgaatt ccacacagca gtttgtgact aatagttgtc ttgccagccc 1080

aggaaagtgg cccacaggtc aggccatccc gtgggacaca ggatgaattt ttcttctctg 1140

ggtcattgtc atgtcagacc cctattcact tcagtaggga tggcaccagg ttcaagaggc 1200

caaagaagag atggagtcag caaacaaaca taggttttac tgggggaatc tgtttacagg 1260

gagatccagc agcagtgggc tggacaggag aacaacaact actggtaaaa acaaatgcag 1320

ttaattttca ctttgcaccc tccctgcagc aacctccacg tggcaacttt atttcttaag 1380

ttattgctct caggtgcaca ccatacagtt attgagagca gtgctcagaa aggtcagtcc 1440

tgggtcaagg tctcccttct cctgagaagg gattgggcat caaactcttg aagagagaga 1500

gcaagaacat agatattaag tcacatttcc tttgtcttcc aacaggccaa gggctcagag 1560

gcttacaggg cccccctgga aagttggggc ctccaggaaa tccagggcct tctgggtcac 1620

caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 45
<211> 1802
<212> DNA
<213> Homo sapiens

<400> 45
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatggcacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080

aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 46
<211> 1802
<212> DNA
<213> Homo sapiens

<400> 46
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540

gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatgacacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 47
<211> 1802
<212> DNA
<213> Homo sapiens

<400> 47

gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatgccacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740

tcctgttcct tcaaaggaag atacccaaat ttgctttctg acccagtgcc ctcagccctc 1800
tc 1802

<210> 48
<211> 1802
<212> DNA
<213> Homo sapiens

<400> 48
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatgtcacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200

caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 49
<211> 1802
<212> DNA
<213> Homo sapiens

<400> 49
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660

gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggcgt gatgncacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 50
<211> 1802
<212> DNA
<213> Homo sapiens

<400> 50
gaattcctgc cagaaagtag agaggtattt agcactctgc cagggccaac gtagtaagaa 60
atttccagag aaaatgctta cccaggcaag cctgtntaaa acaccaaggg gaagcaaact 120

ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggtgt gatgncacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 51
<211> 1802
<212> DNA
<213> Homo sapiens

<400> 51
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggagt gatgncacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320

ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 52
<211> 1802
<212> DNA
<213> Homo sapiens

<400> 52
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780

gtgacctgtg aggatgccca aaagacctgc cctgcagtga ttgcctgtag ctctccaggc 840
atcaacggct tcccaggcaa agatgggggt gatgncacca aggnagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 53
<211> 1802
<212> DNA
<213> Homo sapiens

<400> 53
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240

gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatgncacca agggagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 54

<211> 1802

<212> DNA

<213> Homo sapiens

<400> 54
   gaattcctgc cagaaagtag agaggtattt agcactctgc cagggccaac gtagtaagaa 60
   atttccagag aaaatgctta cccaggcaag cctgtntaaa acaccaaggg gaagcaaact 120
   ccagttaatt ctgggctggg ttggtgacta aggttgaggt tgatctgagg ttgagacctt 180
   cctctttgga tcaccagctt tcagctcagg gcctgccaat gagtaaatga tagttaacag 240
   gtcctggagg ggaatcagct gcccagatac aaagatggga ttcaggtggc agatggaccc 300
   gaagaggaca tggagagaaa gaggaagctc ctacagacac ctgggtttcc actcattctc 360
   attccctaag ctaacaggca taagccagct ggcaatgcac ggtcccattt gttctcactg 420
   ccacngaaag catgtttata gtcttccagc agcaacgcca ggtgtctagg cacagatgaa 480
   cccctcctta ggatccccac tgctcatcat agtgcctacc tttgttaaag tactagtcac 540
   gcagtgtcac aaggaatgtt tacttttcca aatccccagc tagaggccag ggatgggtca 600
   tctatttcta tatagcctgc acccagattg taggacagag ggcatgctng gtaaatatgt 660
   gttcattaac tgagattaac cttccctgag ttttctcaca ccaaggtgag gaccatgtcc 720
   ctgtttccat cactccctct ccttctcctg agtatggtgg cagcgtctta ctcagaaact 780
   gtgacctgtg aggatgccca aaagacctgc cctgcagtga ttgcctgtag ctctccaggc 840
   atcaacggct tcccaggcaa agatgggngt gatgncacca aggaagaaaa gggggaacca 900
   ggtacgtgtt gggctgttct gtctctgcaa ttctttacct tccagaggaa actgcctggg 960
   gatatgagga gactgatgtc ctatttgagt atatttttct caactatact gtaactcaaa 1020
   acagagattc agctcgaatt ccacacagca gtttgtgact aatagttgtc ttgccagccc 1080
   aggaaagtgg cccacaggtc aggccatccc gtgggacaca ggatgaattt ttcttctctg 1140
   ggtcattgtc atgtcagacc cctattcact tcagtaggga tggcaccagg ttcaagaggc 1200
   caaagaagag atggagtcag caaacaaaca taggttttac tgggggaatc tgtttacagg 1260
   gagatccagc agcagtgggc tggacaggag aacaacaact actggtaaaa acaaatgcag 1320
   ttaattttca ctttgcaccc tccctgcagc aacctccacg tggcaacttt atttcttaag 1380
   ttattgctct caggtgcaca ccatacagtt attgagagca gtgctcagaa aggtcagtcc 1440
   tgggtcaagg tctcccttct cctgagaagg gattgggcat caaactcttg aagagagaga 1500
   gcaagaacat agatattaag tcacatttcc tttgtcttcc aacaggccaa gggctcagag 1560
   gcttacaggg cccccctgga aagttggggc ctccaggaaa tccagggcct tctgggtcac 1620
   caggaccaaa gggccaaaaa ggagaccctg gaaaaagtcc gggtaaggac cccagcaagg 1680
   tctgagctga cttcacccag ggttctgaga ccttgagtat ctggtaagag gtgccccttc 1740
   tcctgttcct tcaaaggaag atacccaaat ttgctttctg acccagtgcc ctcagccctc 1800
   tc 1802

<210> 55
<211> 1802
<212> DNA
<213> Homo sapiens

<400> 55
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360
attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatgncacca aggtagaaaa gggggaacca 900

ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 56
<211> 1802
<212> DNA
<213> Homo sapiens

<400> 56
gaattcctgc cagaaagtag agaggtattt agcactctgc cagggccaac gtagtaagaa 60
atttccagag aaaatgctta cccaggcaag cctgtntaaa acaccaaggg gaagcaaact 120
ccagttaatt ctgggctggg ttggtgacta aggttgaggt tgatctgagg ttgagacctt 180
cctctttgga tcaccagctt tcagctcagg gcctgccaat gagtaaatga tagttaacag 240
gtcctggagg ggaatcagct gcccagatac aaagatggga ttcaggtggc agatggaccc 300
gaagaggaca tggagagaaa gaggaagctc ctacagacac ctgggtttcc actcattctc 360

attccctaag ctaacaggca taagccagct ggcaatgcac ggtcccattt gttctcactg 420
ccacngaaag catgtttata gtcttccagc agcaacgcca ggtgtctagg cacagatgaa 480
cccctcctta ggatccccac tgctcatcat agtgcctacc tttgttaaag tactagtcac 540
gcagtgtcac aaggaatgtt tacttttcca aatccccagc tagaggccag ggatgggtca 600
tctatttcta tatagcctgc acccagattg taggacagag ggcatgctng gtaaatatgt 660
gttcattaac tgagattaac cttccctgag ttttctcaca ccaaggtgag gaccatgtcc 720
ctgtttccat cactccctct ccttctcctg agtatggtgg cagcgtctta ctcagaaact 780
gtgacctgtg aggatgccca aaagacctgc cctgcagtga ttgcctgtag ctctccaggc 840
atcaacggct tcccaggcaa agatgggngt gatgncacca aggcagaaaa gggggaacca 900
ggtacgtgtt gggctgttct gtctctgcaa ttctttacct tccagaggaa actgcctggg 960
gatatgagga gactgatgtc ctatttgagt atatttttct caactatact gtaactcaaa 1020
acagagattc agctcgaatt ccacacagca gtttgtgact aatagttgtc ttgccagccc 1080
aggaaagtgg cccacaggtc aggccatccc gtgggacaca ggatgaattt ttcttctctg 1140
ggtcattgtc atgtcagacc cctattcact tcagtaggga tggcaccagg ttcaagaggc 1200
caaagaagag atggagtcag caaacaaaca taggttttac tgggggaatc tgtttacagg 1260
gagatccagc agcagtgggc tggacaggag aacaacaact actggtaaaa acaaatgcag 1320
ttaattttca ctttgcaccc tccctgcagc aacctccacg tggcaacttt atttcttaag 1380
ttattgctct caggtgcaca ccatacagtt attgagagca gtgctcagaa aggtcagtcc 1440
tgggtcaagg tctcccttct cctgagaagg gattgggcat caaactcttg aagagagaga 1500
gcaagaacat agatattaag tcacatttcc tttgtcttcc aacaggccaa gggctcagag 1560
gcttacaggg cccccctgga aagttggggc ctccaggaaa tccagggcct tctgggtcac 1620
caggaccaaa gggccaaaaa ggagaccctg gaaaaagtcc gggtaaggac cccagcaagg 1680
tctgagctga cttcacccag ggttctgaga ccttgagtat ctggtaagag gtgccccttc 1740
tcctgttcct tcaaaggaag atacccaaat ttgctttctg acccagtgcc ctcagccctc 1800
tc 1802

<210> 57
<211> 18
<212> DNA

<213> Homo sapiens

<400> 57
ngtgatggca ccaaggna                                                    18

<210> 58
<211> 18
<212> DNA
<213> Homo sapiens

<400> 58

ngtgatgaca ccaaggna                                                    18

<210> 59
<211> 18
<212> DNA
<213> Homo sapiens

<400> 59
ngtgatgtca ccaaggna                                                    18

<210> 60
<211> 18
<212> DNA
<213> Homo sapiens

<400> 60
ngtgatgcca ccaaggna                                                    18

<210> 61
<211> 18
<212> DNA
<213> Homo sapiens

<400> 61
ggtgatgnca ccaaggna 18

<210> 62
<211> 18
<212> DNA
<213> Homo sapiens

<400> 62
agtgatgnca ccaaggna 18

<210> 63
<211> 18
<212> DNA
<213> Homo sapiens

<400> 63
tgtgatgnca ccaaggna 18

<210> 64
<211> 18
<212> DNA
<213> Homo sapiens

<400> 64
cgtgatgnca ccaaggna                                                    18

<210> 65
<211> 18
<212> DNA
<213> Homo sapiens

<400> 65
ngtgatgnca ccaaggga                                                    18

<210> 66
<211> 18
<212> DNA
<213> Homo sapiens

<400> 66
ngtgatgnca ccaaggaa                                                    18

<210> 67
<211> 18
<212> DNA
<213> Homo sapiens

<400> 67
ngtgatgnca ccaaggta                                                    18

<210> 68

<211> 18

<212> DNA

<213> Homo sapiens

<400> 68

ngtgatgnca ccaaggca 18

<210> 69

<211> 30

<212> DNA

<213> Homo sapiens

<400> 69

acacacccgt ttccaccctg gagaggccag 30

<210> 70

<211> 30

<212> DNA

<213> Homo sapiens

<400> 70

tgcgcagtgc tggagtgcgg cctccgctct 30

<210> 71

<211> 19

<212> DNA

<213> Homo sapiens

<400> 71
cctgtgagga actactgtc                                                    19

<210> 72
<211> 20
<212> DNA
<213> Homo sapiens

<400> 72
ggtgcacggt ctacgagacc                                                   20

## Description

### Technical Field

The present invention relates to methods for obtaining information about nucleic acid associated with disease obtained from an individual. The present invention relates to methods for obtaining information about nucleic acid of an individual as well as information about nucleic acid obtained about the disease, and in particular, information about the nucleic acid of a pathogenic microorganism associated with disease. The present invention also provides probe-immobilized substrates, and in particular, probe-immobilized chips for use in the methods.

### Background Art

Techniques for detecting genes by using a DNA chip are disclosed in (Beattie et al., Clin Chem 39: 719-22, Fodor et al., Science, 251, 767 (1991), Khrapko et al., FEBS Lett, 256, 118 (1989), and Southern et al., Nucleic Acids Res., 22, 1368 (1994)). A DNA chip is a glass or silicon substrate of several centimeters square having a plurality of DNA probes different in sequence immobilized thereon. The DNA probes immobilized on such a chip are reacted with a test nucleic acid labeled with a marker such as a fluorescent pigment or a radioisotope (RI) or a mixture containing an unlabeled test nucleic acid and a labeled oligonucleotide. When a sequence complementary to a DNA probe immobilized on the chip is present in a sample, a signal derived from the label attached to a specific site of the chip is obtained. Therefore, if the sequence and the position of an immobilized DNA probe are known in advance, the base sequence present in the test nucleic acid. can be identified (Pease et al., Proc. Natl. Acad. Sci. USA, 91, 5022 (1994) Parinov et al., Nucleic Acids Res., 24, 2998 (1996).

By obtaining a specimen, such as blood, from a patient, and extracting the nucleic acid from the specimen, a nucleic acid intrinsic to the patient can be obtained. If the nucleic acid intrinsic to the patient is further subjected to gene analysis, information on a constitutional predisposition of the patient, such as the susceptibility of the patient to disease, the efficacy of a medicinal agent, and/or the occurrence of side effects can be obtained.. As a result, it is possible to obtain patient-specific information. Treatments designed to be specific to individual patients is called "Tailor-made medical care (Customized medical care)".

There remains a need to develop methods for proposing medical treatment suitable for a disease more accurately and easily on the basis of a specimen taken from the patient.

All references and patent publications disclosed herein are hereby incorporated by reference in their entirety.

### Disclosure of Invention

A method for obtaining first information about a nucleic acid of an individual subject to a specific disease, and second information about a nucleic acid from a pathogenic microorganism present in said individual wherein said pathogenic organism is associated with the specific disease, comprising:
(a) reacting an extract of nucleic acid from the individual with a probe-immobilized substrate comprising a first probe and a second probe, wherein said first probe detects the presence of a specific nucleic acid sequence of the pathogenic microorganism, wherein said pathogenic microorganism is associated with said specific disease, and wherein said second probe detects the presence of a specific nucleic acid sequence of said individual; and
(b) obtaining said first information by detecting the presence of nucleic acid bound to said first probe, if any, and obtaining said second information by detecting the presence of nucleic acid bound to said second probe, if any, resulting from said reaction of (a).

### Brief Description of Drawings

FIG. 1 is a schematic view of a DNA chip according to an embodiment;
FIG. 2 is a schematic view of a PNA chip according to an embodiment; and
FIG. 3 is a flowchart showing a method according to an embodiment.

### Best Mode for Carrying Out of the Invention

The present invention relates to methods of obtaining information about nucleic acid from an individual subject to a disease. The individual may be in early or late stages of the disease. The individual may be a subject who may have already suffered from or will suffer from the disease.

The present invention relates to the correlation of nucleic acid information from both the individual and the disease. In embodiments disclosed herein, the individual is subject to a disease associated with a pathogenic microorganism, such as for example a virus, a bacteria, a yeast or a mycoplasma, and the present invention relates to the correlation of nucleic acid information obtained from the pathogenic microorganism present in the individual. As well as nucleic acid from the individual.

A Accordingly, in one aspect, the present invention provides methods for obtaining information about a nucleic acid associated with a specific disease of an individual, and in particular, nucleic acid that is associated with responsiveness to a treatment of the disease of the individual, as well as information about a nucleic acid associated with the specific disease derived from a pathogenic microorganism present in the individual. The present invention also relates to specific nucleic acid probes used in the identification of nucleic acid from the individual and from the pathogenic microorganism. In some embodiments, the nucleic acid probes are immobilized on a substrate, such as a chip. The present invention also relates to probe immobilized substrates, such as probe-immobilized chips, for use in the methods. The other substrates such as DNA capillary electrophoresis device, some kinds of beads, membrane-based array, microtiter plate, electrode, some kinds of devise based on the semiconductor, some kinds of wave guide materials, Surface plasmon resonance (SPR), Quartz crystal microbalance (QCM), and Mass-spectroscopy are also used for the present invention. Furthermore, allele-specific oligohybridization, restriction methods (microtitierarray diagonal gel electrophoresis), ligation methods (padlock probe, oligonucreoride ligation assay, dye-labeled oligonucreoride ligation), nucleotide incorporation (minisequencing, template-directed dye-termination assay) and invader assay are also used for the present invention. Other general hybridization techniques in solution are also used for the present invention such as amplification method (polymerase chain reaction (PCR), ligase chain reaction (LCR), nucleic acid sequence-based amplification (NASBA), strand displacement amplification (SDA), loop-mediated isothermal amplification (LAMP), isothermal and chimeric primer-initiated amplification of nucleic acids (ICAN), branched DNA) which could be used to detect nucleic acid of the individual and pathogenic organism.

Samples obtained from an individual that contain nucleic acid include whole blood, blood, serum, leukocytes, urine, feces, seminal fluid, saliva, biopsy samples, cultured cells, and sputum. A preferable specimen is blood. The specimen preferably contains both a nucleic acid derived from an individual that is associated with responsiveness to a treatment for a disease and a nucleic acid of a pathogenic microorganism present in the individual, wherein the pathogenic microorganism is associated with the disease. If necessary, the specimen may be subjected to a pretreatment such as homogenization and extraction. The pretreatment may be appropriately chosen by one skilled in the art depending upon a type of specimen.

In one illustrative embodiment disclosed herein, a whole blood sample is first taken from a human and nucleic acid derived from the human genome and nucleic acid derived from a viral genome present within the human are taken from the whole blood sample by use of a commercially available human genome extraction kit, for example, a QIAamp DNA Blood Midi kit (Trade name, manufactured by QIAGEN, Tokyo, Japan). In some embodiments, the nucleic acids that may contain a target sequence are amplified. Thereafter, the amplified product thus obtained is analyzed by hybridization to a probe-immobilized chip followed by detection of nucleic acid that binds to the chip. In this way, information about a nucleic acid associated with a specific disease is obtained from the individual together with information about a nucleic acid associated with the specific disease obtained from a pathogenic microorganism present in the individual.

In the aforementioned method, as an example of the individual, a human is used. However, the individual is not limited to a human. According to an aspect of the present invention, the "individual" may be any vertebrate, including mammals such as primates and including humans, dogs, cats, cows, goats, pigs, sheep, and monkeys. Among them, a human is the most suitable individual. According to an aspect of the present invention, the individual may be healthy or suffering from a disease. However, in general, methods of the present invention can be more effectively used if applied to a human who suffers from a disease caused by a pathogenic microorganism and a type of a certain nucleic acid sequence in the individual. The term "specific disease" used herein encompasses diseases associated with a pathogenic microorganism, such as for example, a virus, bacterium, yeast or mycoplasma as well as oncological diseases, and preferably refers to a disease associated with a pathogenic microorganism, and more preferably, to a disease whose occurrence and whose therapeutic effect are varied depending upon a type of nucleic acid contained in an individual suffering from the disease and/or the presence and absence of the expression of a gene having such a nucleic acid. However, the disease is not particularly limited.

The term "target nucleic acid" used herein refers to a nucleic acid containing the sequence to be detected and/or analyzed.

According to an aspect of the present invention, as the nucleic acid derived from an individual to be extracted, any nucleic acid may be used as long as it is derived from an individual. More specifically, the nucleic acid may be any one of DNA, RNA, and a genomic DNA. As the nucleic acid derived from a pathogenic microorganism to be extracted, any nucleic acid may be used as long as it is derived from a target pathogenic microorganism, and more specifically, DNA or RNA.

A method for extracting a nucleic acid component from a specimen is not limited to the aforementioned means. A liquid-liquid extraction method using phenol-chloroform, a solid-liquid extraction method using a carrier, and the like may be used. Alternatively, a commercially available kit for nucleic acid extraction called QIAamp (manufactured by QIAGEN), a Genomic DNA purification kit(manufactured by Promega) or an SMAI test (manufactured by Sumitomo Metal Co., Ltd) may be used but not limited thereto. The nucleic acid thus extracted may be or may not be subjected to amplification such as PCR.

In the case wherein the extracted nucleic acid component is RNA, the RNA may be directly used as a test nucleic acid. Alternatively, cDNA prepared from RNA by using a reverse transcriptase may be used as a test nucleic acid. In this case, a nucleic acid derived from an individual and a nucleic acid derived from a pathogenic microorganism may be used in mixture without isolating them. More specifically, these nucleic acids are extracted by using the QIAamp DNA Blood Midi Kit in accordance with the aforementioned method, treated with a reverse transcriptase, and subjected to PCR amplification.

According to an aspect of the present invention, amplification may be performed by any known amplification means as long as it is generally used for amplifying a nucleic acid. Amplification is preferably performed by a polymerase chain reaction (hereinafter referred to as a "PCR"). The amplification step may be omitted if a specimen contains a sufficient amount of nucleic acids.

According to an aspect of the present invention, a probe-immobilized substrate, such as a probe-immobilized chip comprises a first probe and a second probe having different base sequences and immobilized on a substrate. The first probe is used for detecting the presence of a specific nucleic acid sequence derived from a pathogenic microorganism associated with a specific disease. The second probe is used for detecting the presence of a specific nucleic acid derived from an individual.

Such a probe-immobilized substrate is particularly advantageous because nucleic acids derived from two different origins, such as an individual and a pathogenic microorganism, can be simultaneously analyzed by using the same substrate. Such a probe-immobilized chip is disclosed for the first time by the present inventors. According to the present invention, such a probe-immobilized substrate is provided as one aspect of the present invention.

In the probe-immobilized chip according to an aspect of the present invention, the first probe is used for detecting the presence of a nucleic acid sequence derived from a pathogenic microorganism contained in a specimen, such as for example, a whole blood specimen from a human patient. As such, a probe includes, a probe having the base sequence corresponding to chromosomal DNA or a gene (RNA) derived from a pathogenic microorganism which may infect a target organism (e.g., a human patient). Besides these, the base sequence corresponding to cDNA, cRNA, a fragment of each of the chromosomal DNA, RNA, cDNA, and cRNA, or a complementary sequence of each of the fragments may be used as the first probe.

Furthermore, the amount of a pathogenic microorganism contained in a specimen can be measured by using a first probe, that is, a probe having the base sequence corresponding to a nucleic acid sequence derived from a pathogenic microorganism, a fragment of the nucleic acid sequence, or a complementary sequence of the nucleic acid or the fragment thereof.

The first probe is desirably used for detecting not only the presence of a pathogenic microorganism contained in a specimen but also the presence of a gene mutation of the pathogenic microorganism.

A nucleic acid (RNA) expressed by a pathogenic microorganism in a specimen can be measured by the first probe, that is, a probe having the base sequence corresponding to a nucleic acid sequence derived from a pathogenic microorganism, a fragment of the nucleic acid sequence, or a complementary sequence of the nucleic acid and the fragment thereof. In this case, the probe can detect RNA, cDNA, or cRNA, qualitatively and quantitatively.

In one embodiment wherein the pathogenic microorganism is a virus, since a virus expresses a large amount of specific gene in the growth period, if the expression pattern (quantity, quality) can be measured, it is possible to evaluate the efficacy of a medicinal agent.

According to an aspect of the present invention, the second probe used in the probe-immobilized chip is the base sequence corresponding to a chromosomal DNA sequence or a gene (RNA) derived from an individual organism and regarding a disease. Besides these, the base sequence corresponding to cDNA, cRNA, a fragment of each of the chromosomal DNA, RNA, cDNA and cRNA, or a complementary sequence of each of the fragments may be used as the second probe. The nucleic acid of an individual "associated with a disease" is defined as a nucleic acid sequence present in the chromosome of a cell of an individual and predictive of responsiveness to treatment. More specifically, the nucleic acid sequence is predictive of susceptibility of an individual to disease, the efficacy of a medicinal agent, and/or occurrence of side effects.

Because of the presence of the second probe, it is possible to detect the presence of a specific nucleic acid sequence, the expression amount of a specific gene, and the expression pattern of the gene in an individual.

It is desirable that the first probe and second probe according to the present invention have a sequence of at least 11 base, preferably at least 12 base, more preferably at least 13, 14, 15, 16, 17, 18, 19, 20, 21, 22, 23, 24, 25, 26, 27, 28, 29 and even 30 base. If the length of the base sequence to be immobilized on a substrate is excessively long, it is difficult to distinguish variation of a single base. In contrast, if the base sequence to be immobilized to a substrate is excessively short, it is difficult to determine the base sequence contained in a specimen.

As the first or second probe, an oligonulceotide such as ribonucleic acid (RNA), deoxyribonucleic acid (DNA), peptide nucleic acid (PNA), methylphosphonate nucleic acid, or S-oligo; or a polynulceotide such as cDNA and cRNA may be used.

According to an aspect of the present invention, a probe-immobilized substrate is prepared by immobilizing the first probe and the second probe comprising the aforementioned base sequences to a substrate formed of a base plate, a porous material (Beattie et al., Clin. Chem., 41, 700 (1995)), a micro-titer plate (Kawai et al., Anal. Biochem., 209, 63 (1993)), beads (Mirkin et al., Nature, 382, 607 (1996), a spherical material, a granular material, a magnetic material, or magnetic beads (Miller et al., J. Magnet. Magn. Mater., 225, 138 (2001), DNA capillary electrophoresis chip (Chou et al., Proc. Natl. Acd. Sci., 96, 11 (1999), membrane-based array (Lemieux et al., Molecular Breeding, 4, 277 (1998), some kinds of devise based on the semiconductor (Thewes et al., 2002 IEEE International Solid-State Circuits Conference, 350 (2002), some kinds of wave guide materials (Piunno et al., Anal. Chim. Acta, 288, 205 (1994)), SPR (Nelson et al . , Anal. Chem., 73, 1 (2001), QCM (Ito et al . , Anal. Chim. Acta, 327, 29 (1996), and Mass-spectroscopy (Amexis et al., Proc. Natl. Acd. Sci., 98, 12097 (2001). The quality, size, and shape of a material of the substrate for immobilizing a nucleic acid are not particularly limited. A material having any quality, size, and shape may be used as the substrate as long as it can immobilize a nucleic acid. The sequence of a nucleic acid in a specimen is detected by means of a probe-immobilized substrate, for example, by extracting a nucleic acid component from a specimen taken from an individual to obtain a test nucleic acid, bringing the test nucleic acid into contact with a probe-immobilized substrate, such as a probe-immobilized chip, and detecting the hybridization reaction between the probe on the probe-immobilized substrate and the test nucleic acid.

To detect the hybridization reaction between a probe on a probe-immobilized chip and a nucleic acid component in a specimen, the present invention encompasses (1) a method using a marker substance and (2) a method using an electrochemical means.
(1) In the method using a marker substance, a test nucleic acid is previously labeled with a fluorescent pigment such as FITC, Cy3, Cy5 or rhodamine; an enzyme such as biotin, hapten, oxidase or phosphatase; or an electrochemically active substance such as ferrocene or quinones. Alternatively, an another probe labeled with such a marker substance is used for detecting the hybridization reaction. A plurality of marker substances may be used simultaneously.
(2) In the method of using an electrochemical means, a probe-immobilized chip is used as an electrode by using a conductive material for forming the substrate of the probe-immobilized chip for immobilizing a probe. In this case, other than the aforementioned electrode, an opposite electrode and a reference electrode are used for detecting a hybridization reaction, in the same manner as in a general electrochemical detection method. As such a reference electrode, a general reference electrode such as an electrode formed of silver and silver chloride or an electrode formed of mercury and mercuric chloride may be used. A probe-immobilized chip may be formed by immobilizing probes having different base sequences on different conductive substrates and arranging these conductive substrates on the same base plate. If such a probe-immobilized chip is used, detection can be accurately performed. In this case, it is possible to detect which electrochemical signal is derived from which probe.

In some embodiments, the hybridization reaction between a nucleic acid component extracted from a specimen and a probe immobilized on a probe-immobilized chip is performed as follows. The hybridization reaction is performed in a buffer having an ion strength ranging from 0.01 to 5 and a pH ranging from 5 to 10. The hybridization reaction solution may contain additives such as dextran sulfate serving as a hybridization-accelerating agent, salmon sperm DNA, bovine thymus gland DNA, EDTA, and a surfactant. The nucleic acid component extracted from a specimen is added to the buffer solution and denatured with heat at 90°C or more. A probe-immobilized chip may be inserted immediately after the denaturation or after rapid cooling to 0°C. Alternatively, the hybridization reaction may be performed by supplying the hybridization reaction solution dropwise on a substrate. The reaction may be performed while stirring or shaking the reaction solution to increase the reaction speed. The reaction may be performed at a temperature ranging from 10 to 90°C for the period of about one minute to overnight. After the hybridization reaction, electrodes are taken out and washed with a buffer solution having an ion strength ranging from 0.01 to 5 and a pH ranging from 5 to 10.
(1) In the method using a marker substance, the occurrence of a hybridization reaction is detected by detecting a labeled base sequence or a marker of a secondary probe contained in a specimen by means of a detection device appropriately selected depending upon a type of marker. To be more specific, in the case of a fluorescent label, a fluorescent detector may be used.
(2) In the method using an electrochemical means, detection is performed as follows. After a substrate is washed, a double-strand recognition substance, which is capable of selectively binding to a double stranded portion, is applied to the surface of an electrode. The double strand recognition substance used herein is not particularly limited and may include, for example, bis-intercalators such as hoechst 33258, acridine orange, quinacrine, daunomycin, metallo intercalator, and bisacridine; a trisintercalator; and a polyintercalator. Furthermore, such an intercalator may be previously modified with an electrochemically active metal complex such as ferrocene or viologen. The concentration of such a DNA binding substance varies depending upon the type of intercalator, however generally falls within the range of 1 ng/mL to 1 mg/mL. In this case, a buffer having an ion strength ranging from 0.001 to 5 and a pH ranging from 5 to 10 is used. After the electrode is reacted with a double strand recognition substance, washed, and subjected to electrochemical measurement.

The electrochemical measurement is performed by applying at least a potential at which a double strand recognition substance can react electrochemically, followed by measuring a current derived from the double strand recognition substance. The potential may be applied by sweeping it at a constant rate or in a pulse fashion. Alternatively, a constant potential may be applied. The reaction current is measured while controlling the current and voltage by a device such as a potention stat, a digital multimeter or a function generator. The concentration of a target nucleic acid can be calculated from a calibration curve based on the current value thus obtained.

The base sequence detection apparatus using an electrode comprises a nucleic acid extraction section, a nucleic acid reaction section, a double strand recognition substance reaction section, an electrochemical measurement section, and a washing section.

Another DNA chip for measuring a hybridization reaction in an electrochemical method is disclosed in the following document. Hashimoto et al., reported a sequence specific gene detection with a gold electrode modified with DNA probes and an electrochemically active dye. The anodic current derived from dye relates to the concentration of the target DNA. Wang et al. reported an indicator free electrochemical DNA hybridization biosensor. The biosensor format involves the immobilization of inosine-substituted (guanine-free) probe onto the carbon paste electrode, and a direct chronopotentiometric detection of the duplex formation by the appearance of the guanine oxidation peak of the target. These document is incorporated into this text by reference.

In the electrochemical measurement, it is not necessary to label a specimen with a marker. Since detection can be made by measuring an electric signal, a complicated system as used for detecting fluorescence is not required. Therefore, the size of the electrochemical measurement system can be reduced, if desired.

According to an aspect of the present invention, it is possible to obtain not only information on the nucleic acid sequence of an individual patient regarding a disease but also information on the nucleic acid sequence of a pathogenic organism infecting the patient, from a specimen such as blood taken from the patient, in a simple procedure.

According to an aspect of the present invention, a probe-immobilized substrate is used for estimating an appropriate therapeutic method for an individual subject to a disease. The probe-immobilized substrate comprises a second probe and a first probe immobilized on the same substrate, the second probe being used for obtaining information on a nucleic acid sequence linked to a disease and present in a chromosome in a cell of an individual and the first probe being used for obtaining information on the nucleic acid sequence of a pathogenic microorganism associated with the disease. By virtue of the probe-immobilized substrate, it is possible to obtain information regarding a specific disease and/or prediction of responsiveness of treatment of a specific disease derived from both an individual and a pathogenic microorganism from a specimen taken from the individual, simultaneously in a simple manner.

In some embodiments, the methods and probe-immobilized substrates are used for estimating the efficacy of treatment for Hepatitis C. Another embodiments is that the method and probe-immobilized substrates used for estimating of treatment for AIDS (titer of HIV and mutations). Another embodiments is that the method and probe-immobilized substrates used for estimating of treatment for Hepatitis B (titer of HBV, type and mutations).

The efficacy of treatment for hepatitis C can be estimated by a method according to an aspect of the present invention. This case will be explained below by way of example.

Hepatitis C is known to, for example, progress to cirrhosis and further to develop into liver cancer. As one of the treatments for hepatitis C, it is known to administer interferon (hereinafter referred to as "IFN"). IFN alpha and beta are often used for treatment. However, the efficacy of IFN greatly varies. For example, IFN effectively works on only about 20 to 30% of human patients of Japanese origin. Even when IFN works effectively, in a human patient, IFN has the problem of inducing extremely strong side effect in a human patient. Such a variance in efficacy of IFN is considered to be due to differences in the genotype of a hepatitis C virus (hereinafter referred to as "HCV") infecting a patient (see J. Clin. Microbiol., 34, 2516 (1996)) and the amount of virus, and difference in nucleic acid sequence of the patient infected with the virus. On the basis of comparison of nucleotide and predicted amino sequences of defferent regions of the HCV genome, at least six major genotypes have been reported (Forns et al., J. Clin. Microbiol., 34, 2516 (1996), Andonov et al., J. Clin. Microbiol., 33, 254 (1995). According to an aspect of the present invention, it is possible to estimate the efficacy of IFN against hepatitis C. The term "interferon (IFN)" used herein includes interferon α, β, γ and/or ω. For example, if the type of hepatitis C virus infecting a patient is Ib (the dominant type observed in Japan), IFN is less effective. It means that the concentration of HCV-RNA, HCV-antibody, GOP, and GTP in serum does not decrease by IFN treatment. However, if the type of virus is 2a, the efficacy of IFN is higher. It means that the concentration of HCV-RNA, HCV-antibody, GOP, and GTP in serum decrease by treatment. On the other hand, if the amount of virus is as large as 106 copies/mL or more, the efficacy of IFN is low. Therefore, to check the genotype and amount of virus infecting a patient at a nucleic acid level, the first probe according to the present invention is used.

For example, as a probe for detecting the presence of a nucleic acid sequence of the hepatitis C virus (HCV) in a specimen, the base sequence corresponding to HCV-RNA, a fragment of HCV-RNA, a complementary sequence of HCV-RNA, or a fragment thereof is used.
It is desirable that the probe according to the present invention have a sequence of at least 11 base, preferably at least 12 base, more preferably at least 13, 14, 15, 16, 17, 18, 19, 20, 21, 22, 23, 24, 25, 26, 27, 28, 29 and even 30 base for detection of genotype or mutation of HCV-RNA. On the other hand, the probes longer than at least 30, preferably at least about 40, 50, 60, 70, 80, 90, 100, 200, 500, 1000 base are also preferably for detection of HCV genome. The length of 11-30 are suitable chip format and longer probes than 30 base may suitable for general haybridization format. For example, probes having base sequences corresponding to those represented by SEQ ID NO: SEQ ID NO:1, SEQ ID NO:2, SEQ ID NO:3, and SEQ ID NO:4 and complementary sequences thereof may be used. These sequences are almost conserved in all of the genotypes of HCV (Andonov et al., J. Clin. Microbiol., 33, 254 (1995).

As the first probe of the present invention, it is preferable to use a probe capable of detecting not only the presence of a pathogenic microorganism in a specimen but also a gene mutation of the pathogenic microorganism. In the case of hepatitis C, it has been reported that the efficacy of IFN varies depending upon the presence of a site-specific mutation of the nucleic acid sequence of an HCV virus (Nagayama et al., Hepatology, 31, 745 (2000)). As an example of the first probe for detecting the site-specific mutation of the nucleic acid sequence of an HCV virus contained in a specimen, a probe having a base sequence for determining the amino acid positioned at the 434th, 580th, 938th, 962nd, 1176th or 2774th positions of the poly protein of HCV can be used (described in Hepatology, 31, 745 (2000)). The genomic sequences of HCV-1b from hepatocellular carcinoma (HCC) patients tend to have mutant-type residues more abundantly than those from asymptomatic carriers (ASC). If such a probe is used, the efficacy of IFN against hepatitis C can be accurately estimated.

As described in the above, it has been reported that the efficacy of IFN treatment varies depending upon genotypes of HCV. Therefore, the first probe may be the base sequence corresponding to HCV-RNA, a fragment of HCV-RNA, or a complementary sequence of HCV-RNA or the fragment. More specifically, the first probe may be selected from probes for specifically detecting genotypes of 1 type HCV (SEQ ID NO: 5), 2 type HCV (SEQ ID NO: 6), and 3 typeHCV (SEQ ID NO:7), depending upon the genotype to be detected. In other words, the first probe for detection of HCV may be a nucleic acid having the sequence corresponding to the sequence represented by any one of SEQ ID NO:5, SEQ ID NO:6, and/or SEQ ID NO:7 and complementary sequences thereof.

When a probe capable of detecting a genotype is used, if the probes corresponding to different genotypes are immobilized in the same substrate, detection can be made accurately.

The genotype of a pathogenic microorganism in a specimen can be detected as follows. In one embodiment, the nucleic acid sequence of the pathogenic microorganism is first subjected to a PCR by using a primer specific to the genotype, and then the genotype is detected by using an immobilized universal probe. The universal probe used herein may be generally defined as a probe capable of detecting all hepatitis C viruses regardless their genotypes (Andonov et al., J. Clin. Microbiol., 33, 254 (1995).

In the case of hepatitis C, a PCR is performed by using, as a sense strand, a base sequence represented by SEQ ID NO:8 or SEQ ID NO:9, and using, as an anti-sense strand, a base sequence represented by SEQ ID NO:10 for genotype 1a, SEQ ID NO:11 for genotype 1b, SEQ ID NO:12 for genotype 2a, or SEQ ID NO:13 for genotype 3a. Thereafter, detection is performed by using, as a universal probe, a base sequence represented by SEQ ID NO:15.

The amount of a pathogenic microorganism in a specimen may be measured by using a first probe, that is, a probe having the base sequence corresponding to a nucleic acid sequence derived from the pathogenic microorganism, or the base sequence corresponding to a fragment of the nucleic acid, or a complementary sequence thereof.

It has been reported that the efficacy of IFN treatment varies depending upon the amount of HCV in blood in the case of hepatitis C (J. Clin. Microbiol., 33, 254 (1995)). INF is not effective for the patients who carry HCV in serum over 106copy/mL (Yeh et al., J. Med. Biol., 66, 481 (2002). As a probe for evaluating the amount of virus, a base sequence corresponding to HCV-RNA, a fragment of HCV-RNA, or a complementary sequence of each of HCV-RNA and the fragment thereof may be used. When a nucleic acid in a specimen is labeled with a fluorescent marker, the amount of virus, that is, the concentration of virus, is measured by hybridizing a probe on a probe-immobilized chip with the nucleic acid, followed by measuring the fluorescent intensity of the fluorescent marker. In the case of the electrochemical method, the concentration of a virus is obtained by hybridizing a probe on a probe-immobilized chip with the nucleic acid, followed by measuring a current value derived from the chip.

In the probe-immobilized chip of the present invention, if the aforementioned probe (SEQ ID NO:5, SEQ ID NO:6, SEQ ID NO:7) is used as the first probe, it is possible to obtain information about the genotype of a pathogenic microorganism such as a virus infecting an individual, the amount of the virus, the presence of a site-specific mutation, and the quantity and spiecies of an expressed gene. As a result, if the virus is a 1b genotype, which is dominantly observed in the Japanese patients, IFN works less effectively, whereas IFN works effectively if the virus is a 2a genotype. On the other hand, if the amount of virus is as large as 106 copies/mL or more, the efficacy of IFN treatment can be estimated low. In this way, it is possible to estimate the efficacy of IFN treatment for an individual organism.

The present applicant has reported another method for estimating the efficacy of IFN treatment against hepatitis C in Japanese Patent Application Nos. 2001-62371 and 2001-62372. In the method, the efficacy of IFN treatment is determined by a specific single nucleotide polymorphism (hereinafter referred to as "SNP") present in the promoter region (hereinafter referred to as "MxA promoter region") of a human gene encoding an MxA protein. INF is effective for patients who carry specific type of SNP at MxA promoter region. Therefore, the effectiveness of IFN treatment can be estimated by checking for the presence of the SNP. Accordingly, in one embodiment of the present invention, a second probe for detecting an individual nucleic acid is used that detects a SNP in a MxA promoter region.

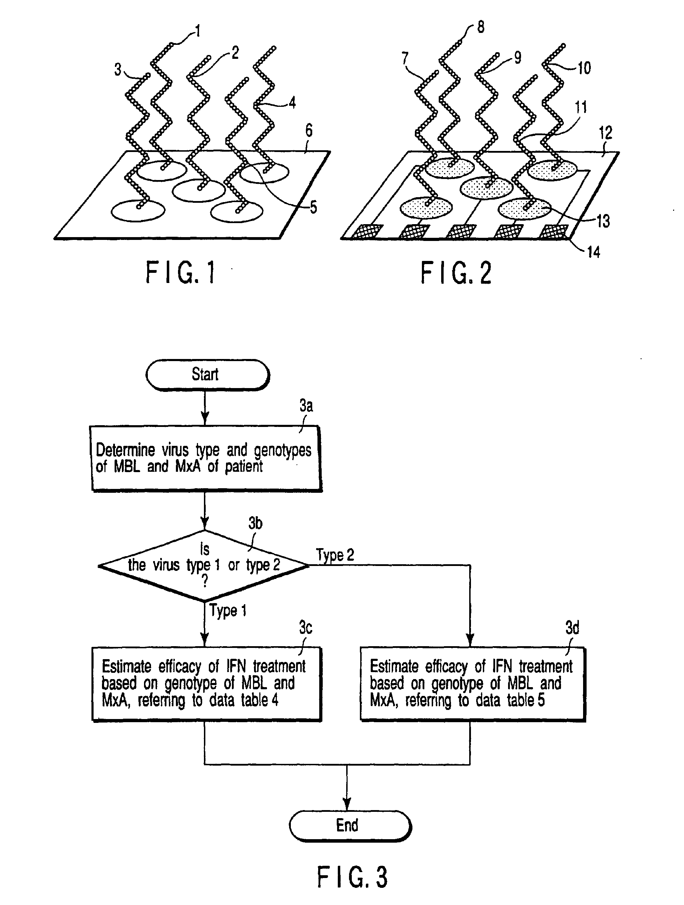

According to the aforementioned finding, the effectiveness of IFN treatment is low in the case the -88th position of the MxA promoter region (hereinafter referred to as "MxA-88") is G/G, whereas the effectiveness is high in the cases of G/T and T/T. On the other hand, the effectiveness of IFN treatment is low in the case the -123rd position of the MxA promoter region (hereinafter referred to as "MxA-123") is C/C, whereas the effectiveness is high in the cases of C/A and A/A. Based on these facts, the effectiveness of IFN treatment is estimated. The terms "the -88 position" and "the -123 position" are relative positions as the transcription initiation site of the MxA gene is regarded as +1. WO 01/71007 Figure 1 shows the nucleotide sequence of the promoter region of the MxA gene.

Therefore, according to an aspect of the present invention, the efficacy of IFN treatment against hepatitis C can be estimated by performing gene analysis for HCV in a specimen taken from an individual and gene analysis for the promoter region of a gene encoding an MxA protein derived from the individual by using a probe-immobilized chip comprising a first probe and a second probe immobilized thereon, where the first probe is used for detecting the presence of an HCV gene and further detecting the genotype of HCV or a gene mutation and the second probe is used for detecting the type of base of SNP appearing in the promoter region of a gene encoding a MxA protein derived from an individual. As a result, the efficacy of IFN treatment is can be easily predicted.

Furthermore, as a method of estimating the efficacy of IFN treatment against hepatitis C, a method for estimating the efficacy of IFN treatment by checking two SNP positions of a gene encoding mannose-binding lectin (hereinafter referred to as "MBL") is known (M. Matsushita et al., J. Hepatology, 29;695-700, 1998). MBL is a key factor of the innate immune system and has genetic polymorphism. A group of MBL genotype "XB" does not respond to IFN. Laursen reported sequences of MBL (Immunology, 93, 421 (1998)). The document is incorporated in this text by reference. The gene analysis for a gene encoding mannose-binding lectin may be performed singly or in combination with the gene diagnosis for the promoter region of a gene encoding an MxA protein.

The MBL polymorphism affecting the efficacy of IFN is present at the -221st position (hereinafter referred to as "MBL-221") of the promoter region of an MBL gene, and at the 52nd, 54th, and 57th codons of exon 1 of the MBL gene. The term "-221st position" is a relative position as the transcription initiation site of the MBL gene is regarded as +1.

C and G are the possible bases to take up the 221st position of the MBL gene. When C takes up the 221st position, the genotype of the MBL gene is designated as "X". When G takes up the 221st position, the genotype is designated as "Y". The nomenclature is based on Madsen et al. (Madsen HO, Garred P, Thiel S, Kurtzhals JAL, Lamm LU, Ryder LP, et al., "The interaction between promoter and structural gene controls the minimum serum level of a human mannan-binding protein", J. Immunol. 1995; 155:3013-20). This document is incorporated in the text by reference.

The codons at the 52nd, 54th and 57th positions of exon 1 of an MBL gene are present in a structural gene, namely, a collagen-like domain. The possible sequence (genotype) of codon 52 is CGT or TGT. The former genotype is classified in type A and the latter genotype is type D according to the Madsen nomenclature. Similarly, the possible sequence (genotype) of codon 54 is GGC or GAC. The former is type A and the latter is type B. Also, the possible sequence (genotype) of codon 57 is GGA or GAA. The former is type A and the latter is type C. In all the codons, type A is a wild-type allele and types B, C and D are mutation-type alleles. The efficacy of IFN is high in the case where all the alleles of codons 52, 54 and 57 are type A compared to other cases where at least one of the alleles of codons 52, 54 and 57 is not type A.

IFN tends to act more effectively on a patient of a YA-YA homo-zygote type whose MBL-211 position is type Y and alleles at codons 52, 54 and 57 are type A, compared to other types of patients, that is, YnonA-YA hetero and YnonA-YnonA homo-zygote types, whose MBL-211 position is type Y and alleles at codons 52, 54 and 57 are not type A. Conversely to say, IFN tends to act less effectively on the patients of YnonA-YA hetero and YnonA-YnonA homo-zygote types than the patient of a YA-YA homo-zygote.

From the aforementioned findings, a proper second probe can be determined. In an embodiment wherein the disease is hepatitis C and the medicinal agent is IFN, a second probe for use in hybridization to a nucleic acid of an individual, including for use on a probe-immobilized chip, is a probe that detects a SNP of the MxA promoter as described infra. In another embodiment wherein the disease is hepatitis C and the medicinal agent is IFN, a second probe for use in hybridization to a nucleic acid of an individual, including for use on a probe-immobilized chip, is a probe that detects an MBL polymorphism that is associated with the efficacy of IFN as described infra.

Promoter regions of a human MxA gene with single nucleotide polymorphism (SNP) present at the 455th position and 420th position of each of these base sequences is related to the efficacy of IFN treatment. Accordingly, the bases sequences represented by SEQ ID NOs:16, 17, 18, 19, 37, 38, 39, and 40, each include a promoter region of a human MxA gene. In further embodiments, a second probe is selected from the group consisting of:
(at455) a base sequence represented by SEQ ID NO:16 as shown in the Sequence Listing;
(bt455) a modified base sequence obtained by modifying the sequence represented by (at455) by deleting or substituting several bases except for the base of the 455th position or adding at least one base thereto;A modified probe for detecting an MBL polymorphism retains the ability to hybridize to a individual nucleic acid encoding MBL. The hybridization conditions using modified probes differ from general probes. It is desirable that the modified probe according to the present invention have a sequence of at least 11 base, preferably at least 12 base, more preferably at least 13, 14, 15, 16, 17, 18, 19, 20, 21, 22, 23, 24, 25, 26, 27, 28, 29 and even 30 base for detection of genotype or mutation of HCV-RNA. On the other hand, the probes longer than at least 30, preferably at least about 40, 50, 60, 70, 80, 90, 100, 200, 500, 1000 base are also preferably for detection of HCV genome. The length of 11-30 are suitable chip format and longer probes than 30 base may suitable for general haybridization format.
(ct455) a base sequence containing bases of the 441st to 455th positions of the sequence represented by SEQ ID NO:16;
(dt455) a base sequence containing bases of the 449th to 459th positions of the sequence represented by SEQ ID NO:16;
(et455) a complementary sequence to a base sequence selected from the base sequences of (at455) to (dt455);
(ag455) a base sequence represented by SEQ ID NO:17 of the list;
(bg455) a modified base sequence obtained by modifying the sequence represented by (ag455) by deleting or substituting several bases except for the base of the 455th position or adding at least one base thereto;
(cg455) a base sequence containing bases of the 441st to 455th positions of the sequence represented by SEQ ID NO:17;
(dg455) a base sequence containing bases of the 449th to 459th positions of the sequence represented by SEQ ID NO:17;
(eg455) a complementary sequence to a base sequence selected from the base sequences of (ag455) to (dg455);
(aa455) a base sequence represented by SEQ ID NO:18 as shown in the Sequence Listing;
(ba455) a modified base sequence obtained by modifying the sequence represented by (aa455) by deleting or substituting several bases except for the base of the 455th position or adding at least one base thereto;
(ca455) a base sequence containing bases of the 441st to 455th positions of the sequence represented by SEQ ID NO:18;
(da455) a base sequence containing bases of the 449th to 459th positions of the sequence represented by SEQ ID NO:18;
(ea455) a complementary sequence to a base sequence selected from the base sequences of (aa455) to (da455);
(ac455) a base sequence represented by SEQ ID NO:19 as shown in the Sequence Listing;
(bc455) a modified base sequence obtained by modifying the sequence represented by (ac455) by deleting or substituting several bases except for the base of the 455th position or adding at least one base thereto;
(cc455) a base sequence containing bases of the 441st to 455th positions of the sequence represented by SEQ ID NO:19;
(dc455) a base sequence containing bases of the 449th to 459th positions of the sequence represented by SEQ ID NO:19; and
(ec455) a complementary sequence to a base sequence selected from the base sequences of (ac455) to (dc455).

The bases sequences represented by SEQ ID NOs:16, 17, 18, 19, 37, 38, 39, and 40, each include a promoter region of a human MxA gene. Single nucleopeptide polymorphism (SNP) present at the 455th position and 420th position of each of these base sequences is related to the efficacy of IFN treatment.

The base sequences of SEQ ID NO:16-19 are identical except for the base of the 455th position. The 455th position of the sequence of SEQ ID NO:16 is thymine. The 455th position of the sequence of SEQ ID NO:17 is guanine. The 455th position of the sequence represented by SEQ ID NO:18 is adenine. The 455th position of the sequence represented by SEQ ID NO:19 is cytosine.

An HCV patient having a nucleic acid sequence represented by SEQ ID NO:16 where the 455th position is thymine is effectively treated with IFN, whereas an HCV patient lacking such a sequence is not effectively treated with IFN. To describe more specifically, an HCV patient (referred to as "G/G homo") having a homologous zygote consisting of the nucleic acid sequences of SEQ ID NO:17 where the 455th position is G is less effectively treated with IFN, compared to an HCV patient (referred to as "G/T hetero") having a heterologous zygote consisting of the nucleic acid sequence of SEQ ID NO: 16 where the 455th position is thymine and the nucleic acid sequence of SEQ ID NO: 17 where the 455th position is guanine, or (and) an HCV patient having a homologous zygote (referred to as "T/T homo") consisting of the nucleic acid sequences of SEQ ID NO:17 where the 455th position is thymine.

Alternatively, compared to an HCV patient of T/non-T hetero or T/T homo, an HCV patient having a homologous zygote (referred to as "nonT/non-T homo") of promoter regions of an MxA gene where the 455 position is not thymine is less effectively treated with IFN. Examples of combinations of non-T/non-T homo are G/G, G/A, G/C, A/A, A/C, A/C, and C/C. Examples of combinations of T/non-T are T/G, T/A, and T/C.

The base sequences represented by SEQ ID NO: 37-40 are identical except for the base of the 425th position. The 425th position of SEQ ID NO: 37 is adenine. The 425th position of the SEQ ID NO:38 is cytosine. The 425th position of SEQ ID NO: 39 is thymine. The 425th position of the SEQ ID NO: 40 is guanine.

In the probe-immobilized chip of the present invention, if the base sequence represented by SEQ ID NO:16 containing an SNP site having thymine, a fragment of the base sequence or any one of complementary sequences (at455) to (et455) is used as a second probe (for detecting the sequence of a nucleic acid derived from an individual), it is possible to know whether the SNP site of the base sequence (including the promoter region of a human MxA gene derived from an individual) is thymine or not before the treatment. In this way, the efficacy of IFN treatment for the individual can be estimated.

If the base of the SNP site of the base sequence including the promoter region of a human MxA gene of an HCV patient is determined prior to the IFN treatment, it is possible to estimate whether IFN effectively acts on the HCV patient.

In the probe-immobilized chip of the present invention, if any one of the base sequence represented by SEQ ID NO:17 containing an SNP site of guanine, a fragment of the base sequence, or any one of complementary sequences (ag455) to (eg455), the base sequence represented by SEQ ID NO:18 containing an SNP site of adenine, a fragment of the base sequence, or any one of complementary sequences (aa455) to (ee455), and the base sequence represented by SEQ ID NO:19 containing an SNP site having a base of adenine, a fragment of the base sequence, or any one of complementary sequences (ac455) to (ec455), is used as a second probe for use in detecting a sequence of a nucleic acid derived from an individual, it is possible to identify the base of the SNP site of the base sequence including the promoter region of a human MxA gene derived from an individual. In this way, the efficacy of IFN treatment for the individual can be estimated (see Japanese Patent Application Nos. 2001-62371 and 2001-62372).

In an embodiment wherein the disease is hepatitis C and the medicinal agent is IFN, a second probe for use in hybridization to a nucleic acid of an individual, including for use on a probe-immobilized chip, is a probe selected from the group consisting of:
(aa420) a base sequence represented by SEQ ID NO:37 of the Sequence Listing;;
(ba420) a modified base sequence obtained by modifying the sequence represented by (aa420) by deleting or substituting several bases except for the base of the 420th position or adding at least one base thereto;
(ca420) a base sequence containing bases of the 415th to 425th positions of the sequence represented by SEQ ID NO:37;
(da420) a complementary sequence to a base sequence selected from the base sequences of (aa420) to (ca420);
(ac420) a base sequence represented by SEQ ID NO:38 of the Sequence Listing;
(bc420) a modified base sequence obtained by modifying the sequence represented by (ac420) by deleting or substituting several bases except for the base of the 420th position or adding at least one base thereto;
(cc420) a base sequence containing bases of the 415th to 425th positions of the sequence represented by SEQ ID NO:38;
(dc420) a complementary sequence to a base sequence selected from the base sequences of (ac420) to (cc420);
(at420) a base sequence represented by SEQ ID NO:39 of the Sequence Listing;
(bt420) a modified base sequence obtained by modifying the sequence represented by (at420) by deleting or substituting several bases except for the base of the 420th position or adding at least one base thereto;
(ct420) a base sequence containing bases of the 415th to 425th positions of the sequence represented by SEQ ID NO:39;
(dt420) a complementary sequence to a base sequence selected from the base sequences of (at420) to (ct420) ;
(ag420) a base sequence represented by SEQ ID NO:40 of the Sequence Listing;
(bg420) a modified base sequence obtained by modifying the sequence represented by (ag420) by deleting or substituting several bases except for the base of the 420th position or adding at least one base thereto;
(cg420) a base sequence containing bases of the 415th to 425th positions of the sequence represented by SEQ ID NO:40; and
(dg420) a complementary sequence to a base sequence selected from the base sequences of (ag420) to (cg420).

An HCV patient having a nucleic acid sequence represented by SEQ ID NO:37 where the 425th position is adenine is effectively treated with IFN, whereas an HCV patient having no such a sequence of No.37 is not effectively treated with IFN. The relationship between the sequence and the efficacy of IFN is almost the same as the case where the SNP site is present at the 455th position.

Furthermore, in the case where a disease which has been confirmed to be effectively treated with IFN is hepatitis C, the second probe of the present invention may be selected from the group consisting of sequences (i) to (t) described below. These sequences (i) to (t) are suitable for determining the types of MBL-221 and presence of polymorphisms of codons 52, 54 and 57.

The sequences of an MBL gene containing MBL-221 and encoding polymorphisms at codons 52, 54 and 57 is shown in SEQ ID NO: 41 to SEQ ID NO:56. MBL-221 is present at the 425th position of these sequences.

Exon 1 starts from the 646th position of each of these sequences. Codon 52 is present from the 868th to 870th position. Codon 54 is from the 874th to 876th position. Codon 57 is the 883rd to 885th position. The SNPs of codons 52, 54, and 57 are present at the 868th position, the 875th position and the 884th position, respectively.
(i) Nucleic acid fragments of SEQ ID NO:41 to SEQ ID NO:44 each containing an SNP site of MBL-221, and each containing the nucleic acid sequence corresponding to the 418th to 432nd position.
(j) Nucleic acid fragments of SEQ ID NO:41 to SEQ ID NO:44 each containing an SNP site of MBL-221 and each containing the nucleic acid sequence corresponding to the 421st to 430th position.
(k) Complementary sequences of the fragments (i) and (j).
(l) Nucleic acid fragments of SEQ ID NO:45 to 56 each containing SNP sites of codons 52, 54 and 57 and each containing the nucleic acid sequence to the 868th to 885th position, for example, fragments containing nucleic acids represented by SEQ ID NO:45 to SEQ ID NO:56.
(m) Nucleic acid fragments of SEQ ID NO:45 to 56 each containing SNP sites of codons 52 and 54 and each containing the nucleic acid sequence corresponding to the 868th to 876th position, for example, fragments containing nucleic acids represented by SEQ ID NO: 45 to SEQ ID NO:56.
(n) Nucleic acid fragments of SEQ ID NO:45 to SEQ ID NO:56 each containing SNP sites of codons 54 and 57, each containing the nucleic acid sequence corresponding to the 874th to 885th position, for example, fragments containing nucleic acids represented by SEQ ID NO: 45 to SEQ ID NO:56.
(o) Nucleic acid fragments of SEQ ID NO:45 to SEQ ID NO:56 each containing an SNP site of codon 54 and each containing the nucleic acid sequence corresponding to the 874th to 876th position, for example, fragments containing nucleic acids represented by SEQ ID NO: 45 to SEQ ID NO:56 and, particularly, fragments containing their 869th to 880th position.
(p) Nucleic acid fragments of SEQ ID NO:45 to SEQ ID NO:56 each containing an SNP site of codon 52 and each containing a nucleic acid corresponding to the 868th to 870th position, for example, fragments containing nucleic acids represented by SEQ ID NO:45 to SEQ ID NO:56 and, particularly, fragments containing the 864th to 873th position of the sequences.
(q) Nucleic acid fragments of SEQ ID NO:45 to SEQ ID NO:56 each containing an SNP site of codon 57 and each containing a nucleic acid corresponding to the 883rd to 885th position, for example, fragments containing nucleic acids represented by SEQ ID NO:45 to SEQ ID NO:56 and, particularly, fragments containing the 880th to 890th position.
(r) Nucleic acid fragments of SEQ ID NO:45 to SEQ ID NO:56 each containing an SNP site of codon 54 and each containing a nucleic acid containing the 875th position where the SNP site of codon 54 is present, for example, fragments containing nucleic acids represented by SEQ ID NO:45 to SEQ ID NO:56.
(s) Nucleic acid fragments represented by (i) to (m) and having a length of 11 to 30 bases. Preferably, for MBL gene, probe sequence contains at least 420th to 430th position, 864th to 874th position, 870th to 880th position and 879th to 889th position. For MxA gene, probe sequence contains at least 450th to 460th.
(t) Nucleic acid fragments represented by (i) to (m), which are modified nucleic acids by deleting, substituting, or adding one to several nucleotides except for the SNP site.

In each of sequences of the Sequence List attached in the text, "N" and "n" represent any one of bases of adenine, thymine, guanine and cytosine.

In the probe-immobilized chip of the present invention, a nucleic acid (RNA) expressed by an individual can be measured by the second probe of the present invention, in other words, by a probe having the base sequence corresponding to a nucleic acid sequence derived from an individual, a fragment thereof, or a complementary sequence of either the nucleic acid sequence or the fragment. In this case, the nucleic acid sequence, which is RNA, cDNA, or cRNA, can be detected or quantified. For example, since the efficacy of IFN varies depending upon a constitutional predisposition of an individual, if a gene expression pattern, that is, the quantity and quality of an expressed gene, can be measured, it is possible to estimate the efficacy of IFN when it is actually administrated.

According to an aspect of the present invention, a target disease is not particularly limited. Any disease may be targeted as long as it can be induced by a pathogenic microorganism. The present invention encompasses diseases associated with a pathogenic microorganism. The present invention also encompasses oncological diseases.

For example, it is possible to predict the efficacy of IFN treatment against diseases other than hepatitis C, known to be treated with IFN effectively, such as for example including but not limited to, infectious diseases caused by infection with virus such as hepatitis C (A, B, C, D, E, F, G types) virus, HIV, influenza virus, herpes virus, adenovirus, human polyomavirus, human papilloma virus, human parbovirus, Mumps virus, human rotavirus, enterovirus, Japanese B Encephalitis virus, dengue virus, rubella virus, and HTLV; and infectious diseases caused by infection with bacteria such as Staphylococcus aureus, hemolytic streptococcus, pathogenic Escherichia coli, enteritis vibrio, Helicobacter pylori, Campylobacter, Vibrio cholerae, dysentery bacilli, salmonellae, Yelsinia, Neisseria gonorrhoeae, Listeria, Leptospira, Legionella, spirochete, Mycoplasma pneumoniae, rickettsiae, chlamydiae, malaria plasmodia, dysentery amoeba and pathogenic fungi; and diseases caused by parasites and Eumycetes.

Furthermore, the efficacy of IFN treatment against oncological diseases can be estimated. Examples of oncological diseases include hereditary diseases, retinoblastoma, Wilm's tumor, familial colonic polyposis, hereditary non polyposis colon cancer, neurofibromatosis, familial chest cancer, xeroderma pigmentosum, blain cancer, oral cancer, esophageal cancer, stomach cancer, colon cancer, liver cancer, pancreatic cancer, lung cancer, thyroid cancer, mammary gland tumor, urinary tumor, virilia tumor, muliebria tumor, skin tumor, osteosarcoma, osteochondrosarcoma, leukemia, lymphoma, and solid tumor.

The pathogenic microorganism to be detected in accordance with the present invention is not particularly limited. For example, a pathogenic microorganism, for example, HCV, may be employed in estimating the efficacy of IFN treatment. However, any pathogenic microorganism may be used as long as it is associated with the disease which has been confirmed to be effectively treated with IFN. A pathogenic microorganism is associated with a disease if it can be directly or indirectly correlated with the disease, or with symptoms of the disease.

Examples of such a pathogenic organism include viruses such as hepatitis C (A, B, C, D, E, F, G types) virus, HIV, influenza virus, herpes virus, adenovirus, human polyomavirus, human papilloma virus, human parbovirus, Mumps virus, human rotavirus, enterovirus, Japanese B Encephalitis virus, dengue virus, rubella virus, and HTLV; and bacteria such as Staphylococcus aureus, hemolytic streptococcus, pathogenic Escherichia coli, enteritis vibrio, Helicobacter pylori, Campylobacter, Vibrio cholerae, dysentery bacilli, salmonellae, Yelsinia, Neisseria gonorrhoeae, Listeria, Leptospira, Legionella, a spirochete, Mycoplasma pneumoniae, rickettsiae, chlamydiae, malaria plasmodia, dysentery amoeba and pathogenic fungi; parasites; and Eumycetes.

The correlation of efficacy of IFN treatment with a disease and a gene intrinsic to an individual has been described by way of example.

However, the present invention is not limited to the example.

In some embodiments, the susceptibility of an individual to acquired immunodeficiency syndrome (AIDS) can be predicted from the analysis of the human immunodeficiency virus (hereinafter, referred to as "HIV") present in the individual and a gene derived from the individual. Furthermore, the genotype of the HIV gene and/or a copy thereof (e.g., cDNA) and a specific polymorphism of the HIV gene are analyzed. Based on the information thus obtained, the efficacy of the following medicinal agents, including a protease inhibitor such as ritnavil (RTV) or saquinavil (SQV) and a reverse transcription inhibitor such as adifothymine (AZT) or didanoisine (ddl) may be predicted. In some embodiments, the susceptibility of an individual to varicella or zona can be predicted by analyzing the Varicella Zoster Virus present in the individual and a gene derived from the individual. Alternatively, the efficacy of an anti-virus agent such as acyclovil can be analyzed. On the other hand, if the genotype of an influenza virus, the copy number of the influenza virus, and the genotype of an individual are determined, it may be possible to predict the susceptibility of the individual to influenza and the efficacy of an anti-virus agent such as Tamifulu. When the nucleic acid component extracted is HCV-RNA, the HCV-RNA may be used as a test nucleic acid. Alternatively, HCV-RNA is converted into cDNA with a reverse transcriptase and thereafter used as a test nucleic acid.

In the case where a probe capable of detecting a genotype is used, if a plurality of probes corresponding to different genotypes may be immobilized on the same substrate, accurate detection can be made. The below examples are intended to illustrate, not limit, the invention.

### EXAMPLES

### Example 1 describes a method for extracting nucleic acid from a human subject.

### 1. Method for extracting human genomic nucleic acid and viral genomic nucleic acid from human patient

About 3 ml of blood was taken from each HCV patient and placed in a vacuum blood collecting tube containing EDTA2K. Thereafter, nucleic acid was extracted from the blood specimen by means of a QIAamp DNA Blood Midi Kit (manufactured and sold by QIAGEN GmbH). HCV genomic RNA contained in the obtained extract was subjected to a reverse transcription reaction. More specifically, an aliquot was taken from the obtained extract, to which a Hexa-deoxyribonucleotide mixture (available from Takara K.K.) and M-MLVReverse Transcriptase (available from Life Technologies Inc.) were used, and a reaction was allowed to proceed at 42°C for 30 minutes.

### 2. Study of extraction rate when HCV genomic RNA is extracted by human genomic nucleic acid extraction kit

When HCV genomic RNA was extracted, serum was generally used in place of whole blood. After contaminants such as proteins had been removed to the greatest extent possible, HCV genomic RNA was extracted by using a guanidine buffer or the like. However, in the method according to the present invention, HCV genomic RNA is extracted simultaneously with human genomic DNA. Thus, the present inventors investigated the extraction rate of HCV genomic RNA when it is extracted simultaneously with human genomic DNA from whole blood by means of a human genomic nucleic acid extraction kit for whole blood.

Two methods were compared. One was a general method of extracting an HCV genomic RNA from serum (100 µL) by using SepeGeneRV-R (available from Sanko Junyaku K. K.). The other was a method of extracting an HCV genomic RNA from whole blood by means of a QIAamp DNA Blood Midi kit (QIAGEN GmbH) which will be referred to simply as the "QIAamp method. The HCV genomic RNA extracted by the QIAamp method was further subjected to a reverse transcriptase reaction as it was and after being purified by ethanol precipitation. The extraction rates of the HCV genomic RNA with and without ethanol precipitation of the QIAamp method were also compared.

The extraction rates were evaluated by a competitive PCR (K. Chayama et al., J. Gastroenterol. Hepatol. 8, S40-44, 1993) using the 5'UTR sequence of HCV genomic RNA. The results are shown in Table 1.

**Table 1**

| Comparison between extraction method and the extraction rate of HCV genomic nucleic acid | | |
|---|---|---|
| Extraction method | Purification | Quantification result (copy/ml in blood) |
| SepeGeneRV-R | Performed | 1 × 10^{5.5} |
| QIAamp DNA Kit | Not performed | 6 × 10^{4.5} |
| QIAamp DNA Kit | Performed | 6 × 10^{4·5} |

From the aforementioned result, it was clear that the extraction rate of HCV genomic RNA was about 10 times lower by the QIAamp method than by the general method. It was therefore presumed that HCV RNA is not extracted by the QIAamp method from a blood specimen having an extremely low viral concentration. Therefore, the QIAamp method was considered unsuitable for the quantitative determination by the probe-immobilized chip of the present invention. However, if a whole blood specimen containing viruses at a concentration of 10 copies/ml or more as measured by the general method is used, it was suggested that, theoretically, DNA could be simultaneously extracted with RNA by the QIAamp method. In other words, it was suggested that viruses are qualitatively determined by the QIAamp method.

The sample obtained by performing a reverse transcription reaction as mentioned above were subjected to an amplification reaction to simultaneously amplify the human genomic nucleic acid and the HCV genomic RNA contained therein.

Primers MxAF01 and MxAR02 were used for amplifying the human genomic nucleic acid. Primer nc2 (K. Chayama et al., J. Gastroenterol. Hapatol. 8, S40-44, 1993, nt27-45) and primer 33 (Okamoto et al., Jpn. J. Exp. Med 60, 215-222, 1990) were used for amplifying the HCV genomic RNA. Amplification was performed in a single tube. The amplification of the human genomic nucleic acid and HCV genomic RNA was performed by repeating 50 times a cycle consisting of a reaction at 94°C for 30 seconds, a reaction at 55°C for 30 seconds, and a reaction at 2°C for one second. A pretreatment was performed at 94°C for 4 minutes before the PCR and a post-treatment was performed at 72°C for 7 minutes after the PCR.

Sequence of Primer:
MxAF01:5'-ACACACCCGTTTCCACCCTGGAGAGGCCAG-3'
MxAR02:5'-TGCGCAGTGCTGGAGTGCGGCCTCCGCTCT-3'
NC2: 5'-CCT GTG AGG AAC TAG TGT C-3'
33: 5'-GGT GCA CGG TCT ACG AGA CC-3'

As a result, both the human genomic DNA (size: 610bp) and the HCV genomic RNA (size: 300bp) were amplified. From this, it was demonstrated that the process from the extraction step of human genomic DNA and virus genomic RNA to the amplification step of specific regions of the genomic nucleic acids was simultaneously performed in the same manner.

According to the present invention, it is possible to estimate treatment for a disease simply and quickly by using a specimen extracted from an individual.

Furthermore, according to such an embodiment, it is possible to obtain much information from a single specimen taken from an individual. It is therefore possible to prevent the mixing up of specimens that sometimes happens during a sample test. Alternatively, when an extremely hazardous specimen is being handled, it is possible to prevent the spread of contamination from such a hazardous specimen compared to the conventional case. At the same time, the risk of an accident caused by the hazardous specimen can be minimized.

Example 2 describes a DNA chip for estimating the efficacy of IFN treatment In the first place, human chromosomal DNA and HCV-RNA were taken out from the blood specimen of a human patient. A fragment of the human chromosomal DNA of 135 bp was amplified by use of primers represented by SEQ ID NO:20 and SEQ ID NO:21. The HCV-RNA was treated with a reverse transcriptase to prepare cDNA.

The nucleic acid sample obtained in the aforementioned step was labeled with FITC by using a kit manufactured and sold by Phamacia Inc.

FIG. 1 shows a schematic view of a DNA chip according to Example 2. A substrate 2 is a slide glass coated with polylysine. On the substrate 2, second probes and first probes, each being 200 nL, were spotted and dried (in FIG. 1, the first and second probes are shown by reference numerals 1 to 5). In this case, the sequences represented by SEQ ID NO:22, 23, 24, 25 and 26 were used as the second probe. The sequences represented by SEQ ID NO:5, 6, and 7 were used as the first probe. Thereafter, the DNA chip was irradiated with UV rays to immobilize the probes on the substrate 2. The base sequences of SEQ ID NO:22-25 were fragments of the base sequences represented by SEQ ID NO:16-19, respectively, each having the SNP site of the 455th position. The base sequences of SEQ ID NO:5-7 were probes used for detecting the genotypes of HCV viruses, namely, 1, 2, 3 types.

The nucleic acids labeled with FITC were dissolved in a 2xSSC-1mmol/L EDTA solution. The nucleic acids thus prepared were placed in a reaction chamber of 20 µL in volume and covered with the probe-immobilized slid glass. After a reaction was performed at 50°C for 12 hours, the probe-immobilized chip was washed twice with a 0.2xSSC-1 mmol/L EDTA solution. Thereafter, the intensity of fluorescence emitted from the slide glass was measured.

As a result, only a spot of the probe of SEQ ID NO:22 emitted a significant level of fluorescence. As a result, the genotype of MxA-88 derived from a human nucleic acid and contained in the blood specimen was determined as T/T homo type. Furthermore, the virus contained in the blood specimen was determined as type 2 of HCV virus. From the obtained fluorescent intensity, the virus concentration was estimated as 106 copies/mL or less. Based on these facts, it was estimated that IFN would act effectively on the patient from which the specimen was taken.

Example 3 describes a PNR chip for estimating the efficacy of IFN treatment.

PNA chip for estimating the efficacy of IFN treatment

Human chromosomal DNA and HCV-RNA were taken from the blood specimen of a human patient. A fragment of 135 bp of the human chromosomal DNA was amplified by using primers of SEQ ID NOs:20 and 21. On the other hand, the HCV-RNA was treated with a reverse transcriptase to prepare cDNA. A PCR was performed by using the cDNA thus obtained as a template and primers represented by SEQ ID NOs:10, 11, 12, 13, and 14 (they were anti-sense primers).

FIG. 2 shows a schematic view of the PNA chip of Example 3. A probe-immobilized chip is prepared as follows. Ten of gold electrodes 13 were patterned on a glass substrate 12 with connections 14. On the chip, the first PNA probes and second PNA probes, each being 200 nL, (indicated by reference numerals 7-11 in the figure) were spotted on the substrate and left them to stand alone for one hour. In this case, the sequences represented by SEQ ID NOs: 27, 28, 29, 30, and 31 were used as the second PNA probes whose N terminals had been modified with cysteine. The sequences represented by SEQ ID NOs:32, 33, 34, 35 and 36 were used as the first PNA probes. The base sequences of SEQ ID NOs:27-30 are fragments including the sequences represented by SEQ ID NOs:16-19 each having the SNP site of the 455th position of the base sequence. The base sequences of SEQ ID NOs:32-36 are used for detecting the genotypes of HCV viruses, namely 1a, 1b, 2a, 2b, and 3a.

Subsequently, a specimen was dissolved in a 2xSSC-1 mmol/L EDTA solution. The resultant solution was placed in a reaction chamber of 20 µL and covered with the probe-immobilized chip. After a reaction was performed at 50°C for one hour, the probe-immobilized chip was washed with a 0.2xSSC-1 mmol/L EDTA solution. To the chip, 10 µmol/L of Hoechst 33258 solution was added dropwise. A gold electrode 13, an opposite electrode thereto, and a reference electrode had been set on the probe-immobilized chip. An oxidation current from the Hoechst 33258 was measured by applying voltage between the gold electrode and the opposite electrode.

As a result, it was observed that the currents obtained from the gold electrodes 13 on which probes having a sequence represented by SEQ ID NOs:27, 28 and 34 were immobilized were significantly changed. From this, the genotype of MxA-88 derived from the human nucleic acid and contained in the specimen was determined as G/T hetero. Furthermore, the virus contained in the specimen was determined as 2a type. Thus, it was estimated that IFN would effectively act upon the patient from which the specimen has been taken.

### Example 4

According to the present invention, it is possible to estimate the effectiveness of IFN treatment for hepatitis C patient based on an HCV virus type infecting a hepatitis C patient and based on the genotypes of Mxa-88, Mxa-123, MBL-221, and codon 54 of a collagen like domain of MBL.

A method of estimating the efficacy of IFN treatment based on the type of virus and the genotypes of MBL and MxA is explained with reference to the flowchart shown in FIG. 3.

The flowchart for estimating the efficacy of IFN is started from Step 3a, where a specimen such as a blood specimen is taken.from an individual to be treated with IFN. If necessary, the specimen thus taken may be purified by extraction or the like. Subsequently, a virus type and the genotypes of MxA-88, MxA-123, MBL-211 and codon 54 of an MBL collagen like domain are determined.

Next, the estimation process goes to Step 3b. If the virus of type 1 is present in Step 3a, the process goes to Step 3c. If only type 2 is present in Step 3b, the process goes to Step 3d.

In Step 3c, by searching Data Table 2 in which the efficacies of IFN are associated with the genotypes, the efficacies of IFN corresponding to the MBL and MxA genotypes determined in Step 3a are determined. As a result, the efficacy of IFN treatment is estimated. The entire estimation process is thus completed.

In Step 3d, by searching Data Table 3 in which the efficacies of IFN are associated with the genotypes, the efficacies of IFN corresponding to the MBL and MxA genotypes determined in Step 3a are determined. As a result, the efficacy of IFN treatment is estimated. The entire estimation process is thus completed.

The Data Tables used herein contain data of the sensitivity to IFN associated with the genotypes.

Data of the data tables 2 and 3 are listed on Table 2 and 3, respectively. Data Tables 2, 3 and Tables 2, 3 used herein are shown by way of example. Any Data Table and Table may be used as long as they show the correlation between the efficacies of IFN and the genotypes. For example, Table 2 shows the relationship between the efficacy of IFN and the genotypes of a patient suffering from type 1 of HCV virus. On the other hand, Table 3 shows the relationship between the efficacy of IFN and the genotype of viruses. The table to be used may indicate requisite items of data alone. Alternatively, the table may include other items of data in combination with the requisite items of data. In Steps 3c and 3d of Example 4, Tables 2 and 3 are referred to as data tables, which have been prepared in advance by picking up requisite items of data for respective steps. However, Tables 2 and 3 may be combined into one. Alternatively, a Table may indicate items other than the requisite items of data. To describe more specifically, items to be listed in a table may be selected as follows. For example, the table to be used in Step 3c may be sufficient if it includes items of "genotypes" of a patient infected with HCV type 1, "percentages of effective patients" and "percentages of non effective patients". The table to be used in Step 3d may be sufficient if it includes items of "genotypes of HCV patient type 2", "percentages of effective patients" and "percentages of non effective patients". However, the items to be listed in table are not limited to these. A performer may arbitrarily select the items to be listed in table.

The numerical values shown in Tables 2 and 3 indicate the correspondence of the genotype and IFN sensitivity or IFN efficacy. However, the correspondence of the genotype and IFN sensitivity is not necessary to be expressed by the numerical values. The correlation between the genotype and the efficacy of IFN may be expressed by any means, for example, symbols such as ○, ×, and Δ, and score (simplified numerical value) such as 1, 2, 3, 4 and 5, as long as it can substantially express the correlation.

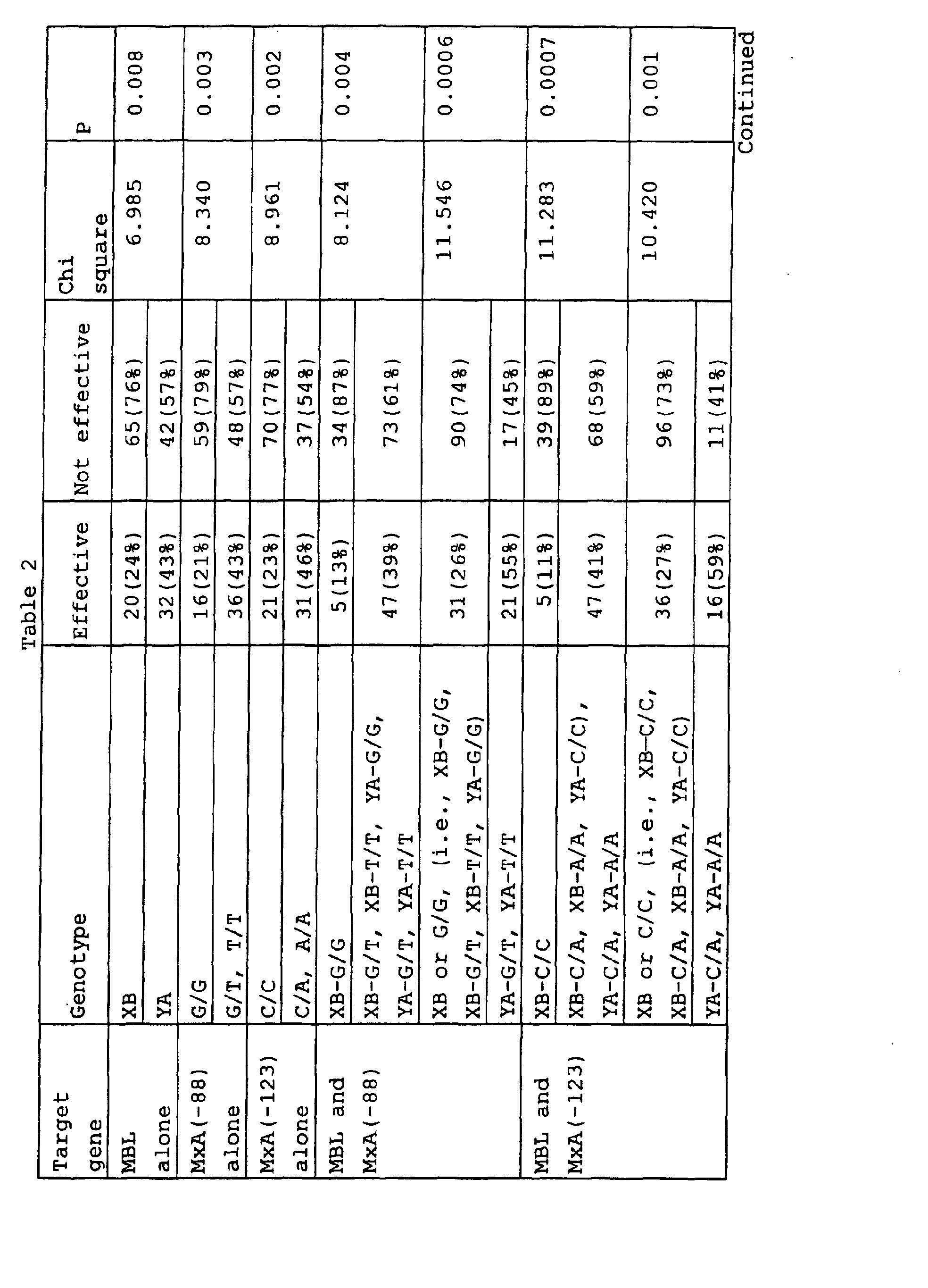

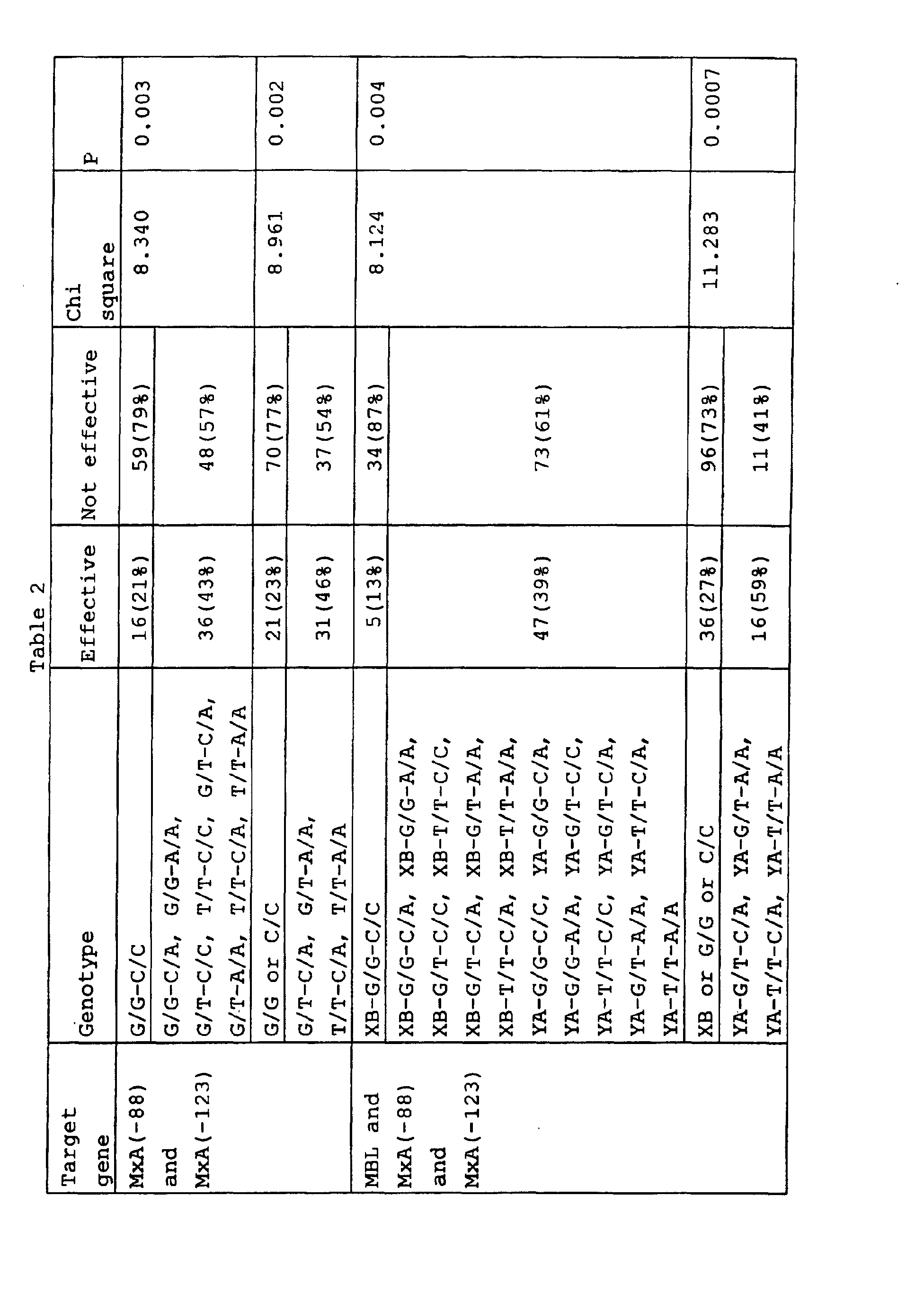

**Table 3**

| Effect of IFN treatment on virus type | | | |
|---|---|---|---|
| HCV type | Effective (%) | Non effective (%) | Total |
| Type 1(b) | 18 (17) | 85(83) | 103 |
| Type 2(a/b) | 34(62) | 21(38) | 55 |
| Type 1&2 | 0 | 1 | 1 |
| Total | 52 | 107 | 159 |

If many items of data are investigated according to the present invention, the efficacy of IFN treatment can be more accurately estimated. Furthermore, if methods and probe-immobilized chip according to aspects of the present invention are used, the information about a target nucleic acid can be collected simply, quickly, and accurately from a specimen. The aforementioned aspect of the present invention is described by way of example and therefore will not limit the present invention.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A method for obtaining first information about a nucleic acid of an individual subject to a specific disease, and second information about a nucleic acid from a pathogenic microorganism present in said individual wherein said pathogenic organism is associated with the specific disease, comprising:
(a) reacting an extract of nucleic acid from the individual with a probe-immobilized substrate comprising a first probe and a second probe, wherein said first probe detects the presence of a specific nucleic acid sequence of the pathogenic microorganism, wherein said pathogenic microorganism is associated withsaid specific disease, and wherein said second probe detects the presence of a specific nucleic acid sequence of said individual; and
(b) obtaining said first information by detecting the presence of nucleic acid bound to said first probe, if any, and obtaining said second information by detecting the presence of nucleic acid bound to said second probe, if any, resulting from said reaction of (a).

2. The method according to claim 1, wherein said nucleic acid of said individual is associated with responsiveness to a treatment for the disease, and wherein the presence of both said nucleic acid from said pathogenic microorganism and said nucleic acid from said individual is correlated with responsiveness to a treatment for the disease.

3. The method of claim 1, further comprising subjecting said extract of nucleic acid from said individual to amplification to obtain amplified nucleic acids prior to reacting in (a).

4. The method according to claim 1, wherein said individual is a human.

5. The method according to claim 1, wherein said extract of nucleic acid is prepared from whole blood.

6. The method according to claim 5, wherein said nucleic acid from an individual is a human genomic nucleic acid; and said nucleic acid from said pathogenic microorganism is a genomic nucleic acid.

7. The method according to claim 1, wherein said nucleic acid from said individual is human genomic nucleic acid, said nucleic acid from said pathogenic microorganism is RNA; and prior to reacting in (a) a reverse transcription reaction is performed.

8. The method according to claim 3, wherein said nucleic acid from said individual is a human genomic nucleic acid, said nucleic acid from said pathogenic microorganism is RNA; and prior to amplification, a reverse transcription reaction is performed.

9. The method according to claim 6, wherein said extract of nucleic acid is obtained by a human genome extraction kit.

10. The method according to claim 9, wherein said human genome extraction kit is QIAamp DNA Blood Midi Kit.

11. The method according to claim 1, wherein said specific disease is hepatitis and said pathogenic microorganism is hepatitis virus.

12. The method according to claim 3, wherein said specific disease is hepatitis and said pathogenic microorganism is hepatitis virus.

13. The method according to claim 11, wherein said first probe detects a genotype of hepatitis C virus and said second probe detects a SNP of the MxA promoter region.

14. The method according to claim 11, wherein said first probe detects a genotype of hepatitis C virus and said second probe detects a polymorphism of the MBL gene.

15. The method according to claim 11, wherein
said first probe comprises at least one type of base sequence selected from the group consisting of:
(a) a base sequence selected from the group consisting of base sequences represented by SEQ ID NO:1, 2, 3 and 5;
(b) a base sequence selected from the group consisting of base sequences represented by SEQ ID NO:5, 6, and 7; and
(c) a complementary sequence to a base sequence selected from (a) and (b), and
said second probe comprises at least one type of base sequence selected from the group consisting of:
(at455) a base sequence represented by SEQ ID NO:16 of the list attached;
(bt455) a modified base sequence obtained by modifying the sequence represented by (at455) by deleting or substituting several bases except for the base of the 455th position or adding at least one base thereto;
(ct455) a base sequence containing bases of the 441st to 455th positions of the sequence represented by SEQ ID NO:16;
(dt455) a base sequence containing bases of the 449th to 459th positions of the sequence represented by SEQ ID NO:16;
(et455) a complementary sequence to a base sequence selected from the base sequences of (at455) to (dt455);
(ag455) a base sequence represented by SEQ ID NO:17 of the list;
(bg455) a modified base sequence obtained by modifying the sequence represented by (ag455) by deleting or substituting several bases except for the base of the 455th position or adding at least one base thereto;
(cg455) a base sequence containing bases of the 441st to 455th positions of the sequence represented by SEQ ID NO:17;
(dg455) a base sequence containing bases of the 449th to 459th positions of the sequence represented by SEQ ID NO:17;
(eg455) a complementary sequence to a base sequence selected from the base sequences of (ag455) to (dg455);
(aa455) a base sequence represented by SEQ ID NO:18 of the list;
(ba455) a modified base sequence obtained by modifying the sequence represented by (aa455) by deleting or substituting several bases except for the base of the 455th position or adding at least one base thereto;
(ca455) a base sequence containing bases of the 441st to 455th positions of the sequence represented by SEQ ID NO:18;
(da455) a base sequence containing bases of the 449th to 459th positions of the sequence represented by SEQ ID NO:18;
(ea455) a complementary sequence to a base sequence selected from the base sequences of (aa455) to (da455);
(ac455) a base sequence represented by SEQ ID NO:19 of the list;
(bc455) a modified base sequence obtained by modifying the sequence represented by (ac455) by deleting or substituting several bases except for the base of the 455th position or adding at least one base thereto;
(cc455) a base sequence containing bases of the 441st to 455th positions of the sequence represented by SEQ ID NO:19;
(dc455) a base sequence containing bases of the 449th to 459th positions of the sequence represented by SEQ ID NO:19;
(ec455) a complementary sequence to a base sequence selected from the base sequences of (ac455) to (dc455);
(aa420) a base sequence represented by SEQ ID NO:37 of the list attached;
(ba420) a modified base sequence obtained by modifying the sequence represented by (aa420) by deleting or substituting several bases except for the base of the 420th position or adding at least one base thereto;
(ca420) a base sequence containing bases of the 415th to 425th positions of the sequence represented by SEQ ID NO:37;
(da420) a complementary sequence to a base sequence selected from the base sequences of (aa420) to (ca420);
(ac420) a base sequence represented by SEQ ID NO:38 of the list;
(bc420) a modified base sequence obtained by modifying the sequence represented by (ac420) by deleting or substituting several bases except for the base of the 420th position or adding at least one base thereto;
(cc420) a base sequence containing bases of the 415th to 425th positions of the sequence represented by SEQ ID NO:38;
(dc420) a complementary sequence to a base sequence selected from the base sequences of (ac420) to (cc420);
(at420) a base sequence represented by SEQ ID NO:39 of the list;
(bt420) a modified base sequence obtained by modifying the sequence represented by (at420) by deleting or substituting several bases except for the base of the 420th position or adding at least one base thereto;
(ct420) a base sequence containing bases of the 415th to 425th positions of the sequence represented by SEQ ID NO:39;
(dt420) a complementary sequence to a base sequence selected from the base sequences of (at420) to (ct420);
(ag420) a base sequence represented by SEQ ID NO:40 of the list attached at a later portion in the text;
(bg420) a modified base sequence obtained by modifying the sequence represented by (ag420) by deleting or substituting several bases except for the base of the 420th position or adding at least one base thereto;
(cg420) a base sequence containing bases of the 415th to 425th positions of the sequence represented by SEQ ID NO:40;
(dg420) a complementary sequence to a base sequence selected from the base sequences of (ag420) to (cg420);
(ag221) a base sequence represented by SEQ ID NO:41 of the list attached at a later portion in the text;
(bg221) a modified base sequence obtained by modifying the sequence represented by (ag221) by deleting or substituting several bases except for the base of the 425th position or adding at least one base thereto;
(cg221) a base sequence containing bases of the 418th to 432nd positions of the sequence represented by SEQ ID NO:41;
(dg221) a base sequence containing bases of the 421st to 430th positions of the sequence represented by SEQ ID NO:41;
(eg221) a complementary sequence to a base sequence selected from the base sequences of (ag221) to (dg221);
(ac221) a base sequence represented by SEQ ID NO:42 of the list attached at a later portion in the text;
(bc221) a modified base sequence obtained by modifying the sequence represented by (ac221) by deleting or substituting several bases except for the base of the 425th position or adding at least one base thereto;
(cc221) a base sequence containing bases of the 418th to 432nd positions of the sequence represented by SEQ ID NO:42;
(dc221) a base sequence containing bases of the 421st to 430th positions of the sequence represented by SEQ ID NO:42;
(ec221) a complementary sequence to a base sequence selected from the base sequences of (ac221) to (dc221);
(aa221) a base sequence represented by SEQ ID NO:43 of the list attached at a later portion in the text;
(ba221) a modified base sequence obtained by modifying the sequence represented by (aa221) by deleting or substituting several bases except for the base of the 425th position or adding at least one base thereto;
(ca221) a base sequence containing bases of the 418th to 432nd positions of the sequence represented by SEQ ID NO:43;
(da221) a base sequence containing bases of the 421st to 430th positions of the sequence represented by SEQ ID NO: 43;
(ea221) a complementary sequence to a base sequence selected from the base sequences of (aa221) to (da221);
(at21) a base sequence represented by SEQ ID NO: 44 of the list attached at a later portion in the text;
(bt221) a modified base sequence obtained by modifying the sequence represented by (at221) by deleting or substituting several bases except for the base of the 425th position or adding at least one base thereto;
(ct221) a base sequence containing bases of the 418th to 432nd positions of the sequence represented by SEQ ID NO:44;
(dt221) a base sequence containing bases of the 421st to 430th positions of the sequence represented by SEQ ID NO:44;
(et221) a complementary sequence to a base sequence selected from the base sequences of (at221) to (dt221);
(ag54) a base sequence represented by SEQ ID NO: 45 of the list attached at a later portion in the text;
(bg54) a modified base sequence obtained by modifying the sequence represented by (ag54) by deleting or substituting several bases except for the base of the 875th position or adding at least one base thereto;
(cg54) a base sequence containing bases of the 874th to 876th positions of the sequence represented by SEQ ID NO: 45;
(dg54) a base sequence containing bases of the 869th to 880th positions of the sequence represented by SEQ ID NO: 45;
(eg54) a complementary sequence to a base sequence selected from the base sequences of (ag54) to (dg54);
(aa54). a base sequence represented by SEQ ID NO: 46 of the list attached at a later portion in the text;
(ba54) a modified base sequence obtained by modifying the sequence represented by (aa54) by deleting or substituting several bases except for the base of the 875th position or adding at least one base thereto;
(ca54) a base sequence containing bases of the 874th to 876th positions of the sequence represented by SEQ ID NO: 46;
(da54) a base sequence containing bases of the 869th to 880th positions of the sequence represented by SEQ ID NO: 46;
(ea54) a complementary sequence to a base sequence selected from the base sequences of (aa54) to (da54);
(ac54) a base sequence represented by SEQ ID NO: 47 of the list attached at a later portion in the text;
(bc54) a modified base sequence obtained by modifying the sequence represented by (ac54) by deleting or substituting several bases except for the base of the 875th position or adding at least one base thereto;
(cc54) a base sequence containing bases of the 874th to 876th positions of the sequence represented by SEQ ID NO: 47;
(dc54) a base sequence containing bases of the 869th to 880th positions of the sequence represented by SEQ ID NO: 47;
(ec54) a complementary sequence to a base sequence selected from the base sequences of (ac54) to (dc54);
(at54) a base sequence represented by SEQ ID NO: 48 of the list attached at a later portion in the text;
(bt54) a modified base sequence obtained by modifying the sequence represented by (ag54) by deleting or substituting several bases except for the base of the 875th position or adding at least one base thereto;
(ct54) a base sequence containing bases of the 874th to 876th positions of the sequence represented by SEQ ID NO:48;
(dt54) a base sequence containing bases of the 869th to 880th positions of the sequence represented by SEQ ID NO:48;
(et54) a complementary sequence to a base sequence selected from the base sequences of (at54) to (dt54);
(ag52-57) a base sequence represented by SEQ ID NO:45 of the list attached at a later portion in the text;
(bg52-57) a modified base sequence obtained by modifying the sequence represented by (ag52-57) by deleting or substituting several bases except for the base of the 875th position or adding at least one base thereto;
(cg52-57) a base sequence containing bases of the 868th to 885th positions of the sequence represented by SEQ ID NO:45;
(dg52-57) a complementary sequence to a base sequence selected from the base sequences of (ag52-57) to (dg52-57);
(aa52-57) a base sequence represented by SEQ ID NO:46 of the list attached at a later portion in the text;
(ba52-57) a modified base sequence obtained by modifying the sequence represented by (aa52-57) by deleting or substituting several bases except for the base of the 875th position or adding at least one base thereto;
(ca52-57) a base sequence containing bases of the 868th to 885th positions of the sequence represented by SEQ ID NO:46;
(da52-57) a complementary sequence to a base sequence selected from the base sequences of (aa52-57) to (ca52-57);
(ac52-57) a base sequence represented by SEQ ID NO:47 of the list attached at a later portion in the text;
(bc52-57) a modified base sequence obtained by modifying the sequence represented by (ac52-57) by deleting or substituting several bases except for the base of the 875th position or adding at least one base thereto;
(cc52-57) a base sequence containing bases of the 868th to 885th positions of the sequence represented by SEQ ID NO:47;
(dc52-57) a complementary sequence to a base sequence selected from the base sequences of (ac52-57) to (cc52-57);
(at52-57) a base sequence represented by SEQ ID NO:48 of the list attached at a later portion in the text;
(bt52-57) a modified base sequence obtained by modifying the sequence represented by (at52-57) by deleting or substituting several bases except for the base of the 875th position or adding at least one base thereto;
(ct52-57) a base sequence containing bases of the 868th to 885th positions of the sequence represented by SEQ ID NO:48; and
(dt52-57) a complementary sequence to a base sequence selected from the base sequences of (at52-57) to (dt52-57).

16. A method according to claim 12, wherein said first probe comprises at least one type of base sequence selected from the group consisting of:
(a) a base sequence selected from the group consisting of base sequences represented by SEQ ID NO:1, 2, 3 and 5;
(b) a base sequence selected from the group consisting of base sequences represented by SEQ ID NO:5, 6, and 7; and
(c) a complementary sequence to a base sequence selected from (a) and (b), and
said second probe comprises at least one type of base sequence selected from the group consisting of:
(at455) a base sequence represented by SEQ ID NO:16 of the list attached;
(bt455) a modified base sequence obtained by modifying the sequence represented by (at455) by deleting or substituting several bases except for the base of the 455th position or adding at least one base thereto;
(ct455) a base sequence containing bases of the 441st to 455th positions of the sequence represented by SEQ ID NO:16;
(dt455) a base sequence containing bases of the 449th to 459th positions of the sequence represented by SEQ ID NO:16;
(et455) a complementary sequence to a base sequence selected from the base sequences of (at455) to (dt455);
(ag455) a base sequence represented by SEQ ID NO:17 of the list;
(bg455) a modified base sequence obtained by modifying the sequence represented by (ag455) by deleting or substituting several bases except for the base of the 455th position or adding at least one base thereto;
(cg455) a base sequence containing bases of the 441st to 455th positions of the sequence represented by SEQ ID NO:17;
(dg455) a base sequence containing bases of the 449th to 459th positions of the sequence represented by SEQ ID NO:17;
(eg455) a complementary sequence to a base sequence selected from the base sequences of (ag455) to (dg455);
(aa455) a base sequence represented by SEQ ID NO:18 of the list;
(ba455) a modified base sequence obtained by modifying the sequence represented by (aa455) by deleting or substituting several bases except for the base of the 455th position or adding at least one base thereto;
(ca455) a base sequence containing bases of the 441st to 455th positions of the sequence represented by SEQ ID NO:18;
(da455) a base sequence containing bases of the 449th to 459th positions of the sequence represented by SEQ ID NO:18;
(ea455) a complementary sequence to a base sequence selected from the base sequences of (aa455) to (da455);
(ac455) a base sequence represented by SEQ ID NO:19 of the list;
(bc455) a modified base sequence obtained by modifying the sequence represented by (ac455) by deleting or substituting several bases except for the base of the 455th position or adding at least one base thereto;
(cc455) a base sequence containing bases of the 441st to 455th positions of the sequence represented by SEQ ID NO:19;
(dc455) a base sequence containing bases of the 449th to 459th positions of the sequence represented by SEQ ID NO:19;
(ec455) a complementary sequence to a base sequence selected from the base sequences of (ac455) to (dc455);
(aa420) a base sequence represented by SEQ ID NO:37 of the list attached;
(ba420) a modified base sequence obtained by modifying the sequence represented by (aa420) by deleting or substituting several bases except for the base of the 420th position or adding at least one base thereto;
(ca420) a base sequence containing bases of the 415th to 425th positions of the sequence represented by SEQ ID NO:37;
(da420) a complementary sequence to a base sequence selected from the base sequences of (aa420) to (ca420);
(ac420) a base sequence represented by SEQ ID NO:38 of the list;
(bc420) a modified base sequence obtained by modifying the sequence represented by (ac420) by deleting or substituting several bases except for the base of the 420th position or adding at least one base thereto;
(cc420) a base sequence containing bases of the 415th to 425th positions of the sequence represented by SEQ ID NO:38;
(dc420) a complementary sequence to a base sequence selected from the base sequences of (ac420) to (cc420);
(at420) a base sequence represented by SEQ ID NO:39 of the list;
(bt420) a modified base sequence obtained by modifying the sequence represented by (at420) by deleting or substituting several bases except for the base of the 420th position or adding at least one base thereto;
(ct420) a base sequence containing bases of the 415th to 425th positions of the sequence represented by SEQ ID NO:39;
(dt420) a complementary sequence to a base sequence selected from the base sequences of (at420) to (ct420) ;
(ag420) a base sequence represented by SEQ ID NO:40 of the list attached at a later portion in the text;
(bg420) a modified base sequence obtained by modifying the sequence represented by (ag420) by deleting or substituting several bases except for the base of the 420th position or adding at least one base thereto;
(cg420) a base sequence containing bases of the 415th to 425th positions of the sequence represented by SEQ ID NO:40;
(dg420) a complementary sequence to a base sequence selected from the base sequences of (ag420) to (cg420);
(ag221) a base sequence represented by SEQ ID NO:41 of the list attached at a later portion in the text;
(bg221) a modified base sequence obtained by modifying the sequence represented by (ag221) by deleting or substituting several bases except for the base of the 425th position or adding at least one base thereto;
(cg221) a base sequence containing bases of the 418th to 432nd positions of the sequence represented by SEQ ID NO:41;
(dg221) a base sequence containing bases of the 421st to 430th positions of the sequence represented by SEQ ID NO:41;
(eg221) a complementary sequence to a base sequence selected from the base sequences of (ag221) to (dg221);
(ac221) a base sequence represented by SEQ ID NO:42 of the list attached at a later portion in the text;
(bc221) a modified base sequence obtained by modifying the sequence represented by (ac221) by deleting or substituting several bases except for the base of the 425th position or adding at least one base thereto;
(cc221) a base sequence containing bases of the 418th to 432nd positions of the sequence represented by SEQ ID NO:42;
(dc221) a base sequence containing bases of the 421st to 430th positions of the sequence represented by SEQ ID NO:42;
(ec221) a complementary sequence to a base sequence selected from the base sequences of (ac221) to (dc221);
(aa221) a base sequence represented by SEQ ID NO:43 of the list attached at a later portion in the text;
(ba221) a modified base sequence obtained by modifying the sequence represented by (aa221) by deleting or substituting several bases except for the base of the 425th position or adding at least one base thereto;
(ca221) a base sequence containing bases of the 418th to 432nd positions of the sequence represented by SEQ ID NO:43;
(da221) a base sequence containing bases of the 421st to 430th positions of the sequence represented by SEQ ID NO:43;
(ea221) a complementary sequence to a base sequence selected from the base sequences of (aa221) to (da221);
(at21) a base sequence represented by SEQ ID NO: 44 of the list attached at a later portion in the text;
(bt221) a modified base sequence obtained by modifying the sequence represented by (at221) by deleting or substituting several bases except for the base of the 425th position or adding at least one base thereto;
(ct221) a base sequence containing bases of the 418th to 432nd positions of the sequence represented by SEQ ID NO:44;
(dt221) a base sequence containing bases of the 421st to 430th positions of the sequence represented by SEQ ID NO:44;
(et221) a complementary sequence to a base sequence selected from the base sequences of (at221) to (dt221);
(ag54) a base sequence represented by SEQ ID NO:45 of the list attached at a later portion in the text;
(bg54) a modified base sequence obtained by modifying the sequence represented by (ag54) by deleting or substituting several bases except for the base of the 875th position or adding at least one base thereto;
(cg54) a base sequence containing bases of the 874th to 876th positions of the sequence represented by SEQ ID NO:45;
(dg54) a base sequence containing bases of the 869th to 880th positions of the sequence represented by SEQ ID NO:45;
(eg54) a complementary sequence to a base sequence selected from the base sequences of (ag54) to (dg54);
(aa54) a base sequence represented by SEQ ID NO: 46 of the list attached at a later portion in the text;
(ba54) a modified base sequence obtained by modifying the sequence represented by (aa54) by deleting or substituting several bases except for the base of the 875th position or adding at least one base thereto;
(ca54) a base sequence containing bases of the 874th to 876th positions of the sequence represented by SEQ ID NO:46;
(da54) a base sequence containing bases of the 869th to 880th positions of the sequence represented by SEQ ID NO:6;
(ea54) a complementary sequence to a base sequence selected from the base sequences of (aa54) to (da54);
(ac54) a base sequence represented by SEQ ID NO: 47 of the list attached at a later portion in the text;
(bc54) a modified base sequence obtained by modifying the sequence represented by (ac54) by deleting or substituting several bases except for the base of the 875th position or adding at least one base thereto;
(cc54) a base sequence containing bases of the 874th to 876th positions of the sequence represented by SEQ ID NO:47;
(dc54) a base sequence containing bases of the 869th to 880th positions of the sequence represented by SEQ ID NO:47;
(ec54) a complementary sequence to a base sequence selected from the base sequences of (ac54) to (dc54);
(at54) a base sequence represented by SEQ ID NO: 48 of the list attached at a later portion in the text;
(bt54) a modified base sequence obtained by modifying the sequence represented by (ag54) by deleting or substituting several bases except for the base of the 875th position or adding at least one base thereto;
(ct54) a base sequence containing bases of the 874th to 876th positions of the sequence represented by SEQ ID NO:48;
(dt54) a base sequence containing bases of the 869th to 880th positions of the sequence represented by SEQ ID NO:48;
(et54) a complementary sequence to a base sequence selected from the base sequences of (at54) to (dt54);
(ag52-57) a base sequence represented by SEQ ID NO:45 of the list attached at a later portion in the text;
(bg52-57) a modified base sequence obtained by modifying the sequence represented by (ag52-57) by deleting or substituting several bases except for the base of the 875th position or adding at least one base thereto;
(cg52-57) a base sequence containing bases of the 868th to 885th positions of the sequence represented by SEQ ID NO:45;
(dg52-57) a complementary sequence to a base sequence selected from the base sequences of (ag52-57) to (dg52-57);
(aa52-57) a base sequence represented by SEQ ID NO:46 of the list attached at a later portion in the text;
(ba52-57) a modified base sequence obtained by modifying the sequence represented by (aa52-57) by deleting or substituting several bases except for the base of the 875th position or adding at least one base thereto;
(ca52-57) a base sequence containing bases of the 868th to 885th positions of the sequence represented by SEQ ID NO:46;
(da52-57) a complementary sequence to a base sequence selected from the base sequences of (aa52-57) to (ca52-57);
(ac52-57) a base sequence represented by SEQ ID NO:47 of the list attached at a later portion in the text;
(bc52-57) a modified base sequence obtained by modifying the sequence represented by (ac52-57) by deleting or substituting several bases except for the base of the 875th position or adding at least one base thereto;
(cc52-57) a base sequence containing bases of the 868th to 885th positions of the sequence represented by SEQ ID NO:47;
(dc52-57) a complementary sequence to a base sequence selected from the base sequences of (ac52-57) to (cc52-57);
(at52-57) a base sequence represented by SEQ ID NO:48 of the list attached at a later portion in the text;
(bt52-57) a modified base sequence obtained by modifying the sequence represented by (at52-57) by deleting or substituting several bases except for the base of the 875th position or adding at least one base thereto;
(ct52-57) a base sequence containing bases of the 868th to 885th positions of the sequence represented by SEQ ID NO:48; and
(dt52-57) a complementary sequence to a base sequence selected from the base sequences of (at52-57) to (dt52-57).

17. The method according to claim 12, wherein said amplification is performed by a PCR using a sense strand represented by at least one base sequence selected from the group consisting of sequences represented by SEQ ID NO:8 and 9, and an anti-sense strand represented by at least one base sequence selected from the group consisting of sequences represented by SEQ ID NO:10, 11, 12, 13 and 14.

18. A probe-immobilized substrate, comprising:
a substrate
a first probe immobilized on the substrate for detecting the presence of a specific nucleic acid of a pathogenic microorganism, if any, wherein said microorganism is associated with a specific disease; and
a second probe immobilized on the substrate and having a different base sequence from that of the first probe, for detecting the presence of a specific nucleic acid of an individual, if any, wherein said nucleic acid of said individual is associated with responsiveness to a treatment for the disease.

19. The probe-immobilized substrate of claim 18, wherein said pathogenic microorganism is hepatitis C virus and said medicinal agent is IFN.

20. The probe-immobilized substrate of claim 18, wherein said nucleic acid of the individual is the promoter region of MxA.

21. The probe-immobilized substrate of claim 18, wherein said nucleic acid of the individual is the gene encoding MBL.

22. The probe-immobilized substrate of claim 18, wherein said first probe detects a genotype of hepatitis C virus.

23. A probe-immobilized substrate comprising:
a substrate
a first probe comprising at least one sequence selected from the group consisting of
(a) a base sequence selected from the group consisting of base sequences represented by SEQ ID NO:1, 2, 3 and 5;
(b) a base sequence selected from the group consisting of base sequences represented by SEQ ID NO:5, 6, and 7; and
(c) a complementary sequence to a base sequence selected from (a) and (b), and
a second probe immobilized on the substrate and comprising at least one sequence selected from the group consisting of:
(at455) a base sequence represented by SEQ ID NO:16;
(bt455) a modified base sequence obtained by modifying the sequence represented by (at455) by deleting or substituting several bases except for the base of the 455th position or adding at least one base thereto;
(ct455) a base sequence containing bases of the 441st to 455th positions of the sequence represented by SEQ ID NO:16;
(dt455) a base sequence containing bases of the 449th to 459th positions of the sequence represented by SEQ ID NO:16;
(et455) a complementary sequence to a base sequence selected from the base sequences of (at455) to (dt455);
(ag455) a base sequence represented by SEQ ID NO:17;
(bg455) a modified base sequence obtained by modifying the sequence represented by (ag455) by deleting or substituting several bases except for the base of the 455th position or adding at least one base thereto;
(cg455) a base sequence containing bases of the 441st to 455th positions of the sequence represented by SEQ ID NO:17;
(dg455) a base sequence containing bases of the 449th to 459th positions of the sequence represented by SEQ ID NO:17;
(eg455) a complementary sequence to a base sequence selected from the base sequences of (ag455) to (dg455);
(aa455) a base sequence represented by SEQ ID NO:18\;
(ba455) a modified base sequence obtained by modifying the sequence represented by (aa455) by deleting or substituting several bases except for the base of the 455th position or adding at least one base thereto;
(ca455) a base sequence containing bases of the 441st to 455th positions of the sequence represented by SEQ ID NO:18;
(da455) a base sequence containing bases of the 449th to 459th positions of the sequence represented by SEQ ID NO:18;
(ea455) a complementary sequence to a base sequence selected from the base sequences of (aa455) to (da455);
(ac455) a base sequence represented by SEQ ID NO:19;
(bc455) a modified base sequence obtained by modifying the sequence represented by (ac455) by deleting or substituting several bases except for the base of the 455th position or adding at least one base thereto;
(cc455) a base sequence containing bases of the 441st to 455th positions of the sequence represented by SEQ ID NO:19;
(dc455) a base sequence containing bases of the 449th to 459th positions of the sequence represented by SEQ ID NO:19;
(ec455) a complementary sequence to a base sequence selected from the base sequences of (ac455) to (dc455);
(aa420) a base sequence represented by SEQ ID NO:37\;
(ba420) a modified base sequence obtained by modifying the sequence represented by (aa420) by deleting or substituting several bases except for the base of the 420th position or adding at least one base thereto;
(ca420) a base sequence containing bases of the 415th to 425th positions of the sequence represented by SEQ ID NO:37;
(da420) a complementary sequence to a base sequence selected from the base sequences of (aa420) to (ca420);
(ac420) a base sequence represented by SEQ ID NO:38;
(bc420) a modified base sequence obtained by modifying the sequence represented by (ac420) by deleting or substituting several bases except for the base of the 420th position or adding at least one base thereto;
(cc420) a base sequence containing bases of the 415th to 425th positions of the sequence represented by SEQ ID NO:38;
(dc420) a complementary sequence to a base sequence selected from the base sequences of (ac420) to (cc420);
(at420) a base sequence represented by SEQ ID NO:39;
(bt420) a modified base sequence obtained by modifying the sequence represented by (at420) by deleting or substituting several bases except for the base of the 420th position or adding at least one base thereto;
(ct420) a base sequence containing bases of the 415th to 425th positions of the sequence represented by SEQ ID NO:39;
(dt420) a complementary sequence to a base sequence selected from the base sequences of (at420) to (ct420);
(ag420) a base sequence represented by SEQ ID NO:40;
(bg420) a modified base sequence obtained by modifying the sequence represented by (ag420) by deleting or substituting several bases except for the base of the 420th position or adding at least one base thereto;
(cg420) a base sequence containing bases of the 415th to 425th positions of the sequence represented by SEQ ID NO:40;
(dg420) a complementary sequence to a base sequence selected from the base sequences of (ag420) to (cg420);
(ag221) a base sequence represented by SEQ ID NO:41 of the list attached at a later portion in the text;
(bg221) a modified base sequence obtained by modifying the sequence represented by (ag221) by deleting or substituting several bases except for the base of the 425th position or adding at least one base thereto;
(cg221) a base sequence containing bases of the 418th to 432nd positions of the sequence represented by SEQ ID NO:41;
(dg221) a base sequence containing bases of the 421st to 430th positions of the sequence represented by SEQ ID NO:41;
(eg221) a complementary sequence to a base sequence selected from the base sequences of (ag221) to (dg221);
(ac221) a base sequence represented by SEQ ID NO:42 of the list attached at a later portion in the text;
(bc221) a modified base sequence obtained by modifying the sequence represented by (ac221) by deleting or substituting several bases except for the base of the 425th position or adding at least one base thereto;
(cc221) a base sequence containing bases of the 418th to 432nd positions of the sequence represented by SEQ ID NO:42;
(dc221) a base sequence containing bases of the 421st to 430th positions of the sequence represented by SEQ ID NO:42;
(ec221) a complementary sequence to a base sequence selected from the base sequences of (ac221) to (dc221);
(aa221) a base sequence represented by SEQ ID NO:43 of the list attached at a later portion in the text;
(ba221) a modified base sequence obtained by modifying the sequence represented by (aa221) by deleting or substituting several bases except for the base of the 425th position or adding at least one base thereto;
(ca221) a base sequence containing bases of the 418th to 432nd positions of the sequence represented by SEQ ID NO:43;
(da221) a base sequence containing bases of the 421st to 430th positions of the sequence represented by SEQ ID NO:43;
(ea221) a complementary sequence to a base sequence selected from the base sequences of (aa221) to (da221);
(at221) a base sequence represented by SEQ ID NO: 44 of the list attached at a later portion in the text;
(bt221) a modified base sequence obtained by modifying the sequence represented by (at221) by deleting or substituting several bases except for the base of the 425th position or adding at least one base thereto;
(ct221) a base sequence containing bases of the 418th to 432nd positions of the sequence represented by SEQ ID NO:44;
(dt221) a base sequence containing bases of the 421st to 430th positions of the sequence represented by SEQ ID NO:44;
(et221) a complementary sequence to a base sequence selected from the base sequences of (at221) to (dt221);
(ag54) a base sequence represented by SEQ ID NO: 45 of the list attached at a later portion in the text;
(bg54) a modified base sequence obtained by modifying the sequence represented by (ag54) by deleting or substituting several bases except for the base of the 875th position or adding at least one base thereto;
(cg54) a base sequence containing bases of the 874th to 876th positions of the sequence represented by SEQ ID NO:45;
(dg54) a base sequence containing bases of the 869th to 880th positions of the sequence represented by SEQ ID NO:45;
(eg54) a complementary sequence to a base sequence selected from the base sequences of (ag54) to (dg54);
(aa54) a base sequence represented by SEQ ID NO: 46 of the list attached at a later portion in the text;
(ba54) a modified base sequence obtained by modifying the sequence represented by (aa54) by deleting or substituting several bases except for the base of the 875th position or adding at least one base thereto;
(ca54) a base sequence containing bases of the 874th to 876th positions of the sequence represented by SEQ ID NO:46;
(da54) a base sequence containing bases of the 869th to 880th positions of the sequence represented by SEQ ID NO:46;
(ea54) a complementary sequence to a base sequence selected from the base sequences of (aa54) to (da54);
(ac54) a base sequence represented by SEQ ID NO: 47 of the list attached at a later portion in the text;
(bc54) a modified base sequence obtained by modifying the sequence represented by (ac54) by deleting or substituting several bases except for the base of the 875th position or adding at least one base thereto;
(cc54) a base sequence containing bases of the 874th to 876th positions of the sequence represented by SEQ ID NO:47;
(dc54) a base sequence containing bases of the 869th to 880th positions of the sequence represented by SEQ ID NO:47;
(ec54) a complementary sequence to a base sequence selected from the base sequences of (ac54) to (dc54);
(at54) a base sequence represented by SEQ ID NO: 48 of the list attached at a later portion in the text;
(bt54) a modified base sequence obtained by modifying the sequence represented by (ag54) by deleting or substituting several bases except for the base of the 875th position or adding at least one base thereto;
(ct54) a base sequence containing bases of the 874th to 876th positions of the sequence represented by SEQ ID NO:48;
(dt54) a base sequence containing bases of the 869th to 880th positions of the sequence represented by SEQ ID NO:48;
(et54) a complementary sequence to a base sequence selected from the base sequences of (at54) to (dt54);
(ag52-57) a base sequence represented by SEQ ID NO:45 of the list attached at a later portion in the text;
(bg52-57) a modified base sequence obtained by modifying the sequence represented by (ag52-57) by deleting or substituting several bases except for the base of the 875th position or adding at least one base thereto;
(cg52-57) a base sequence containing bases of the 868th to 885th positions of the sequence represented by SEQ ID NO:45;
(dg52-57) a complementary sequence to a base sequence selected from the base sequences of (ag52-57) to (dg52-57);
(aa52-57) a base sequence represented by SEQ ID NO:46 of the list attached at a later portion in the text;
(ba52-57) a modified base sequence obtained by modifying the sequence represented by (aa52-57) by deleting or substituting several bases except for the base of the 875th position or adding at least one base thereto;
(ca52-57) a base sequence containing bases of the 868th to 885th positions of the sequence represented by SEQ ID NO:46;
(da52-57) a complementary sequence to a base sequence selected from the base sequences of (aa52-57) to (ca52-57);
(ac52-57) a base sequence represented by SEQ ID NO:47 of the list attached at a later portion in the text;
(bc52-57) a modified base sequence obtained by modifying the sequence represented by (ac52-57) by deleting or substituting several bases except for the base of the 875th position or adding at least one base thereto;
(cc52-57) a base sequence containing bases of the 868th to 885th positions of the sequence represented by SEQ ID NO:47;
(dc52-57) a complementary sequence to a base sequence selected from the base sequences of (ac52-57) to (cc52-57);
(at52-57) a base sequence represented by SEQ ID NO:48 of the list attached at a later portion in the text;
(bt52-57) a modified base sequence obtained by modifying the sequence represented by (at52-57) by deleting or substituting several bases except for the base of the 875th position or adding at least one base thereto;
(ct52-57) a base sequence containing bases of the 868th to 885th positions of the sequence represented by SEQ ID NO:48; and
(dt52-57) a complementary sequence to a base sequence selected from the base sequences of (at52-57) to (dt52-57).

24. The probe-immobilized chip according to claim 18, wherein detection of the presence of the specific nucleic acids is electrochemically performed.

25. The probe-immobilized chip according to claim 23, wherein detection of the presence of the specific nucleic acids is electrochemically performed.

26. A method for determining responsiveness to a treatment for a disease of an individual, wherein the disease is associated with the presence of a pathogenic microorganism in the individual, comprising the steps of:
a) reacting an extract of nucleic acids from said individual with a first nucleic acid probe that hybridizes with nucleic acid of the pathogenic microorganism, wherein the pathogenic microorganism is associated with said disease, and a second nucleic acid probe that hybridizes with target nucleic acid of said individual, wherein the target nucleic acid of said individual is associated with responsiveness to a treatment for a disease of an individual; and
b) detecting said nucleic acid of said pathogenic microorganism that binds to said first nucleic acid probe and said target nucleic acid of said individual that binds to said second nucleic acid probe, wherein the presence of both said nucleic acid of said pathogenic microorganism that binds to said first nucleic acid probe and said target nucleic acid of said individual that binds to said second nucleic acid probe is correlated with responsiveness to a treatment for the disease.

27. The method of claim 26, wherein the probes are immobilized on a substrate.

28. The method of claim 26, further comprising the step of amplifying said nucleic acid from said individual prior to reacting in a).

29. A method for determining susceptibility of an individual to disease, comprising the steps of
a) reacting an extract of nucleic acid from said individual with a first nucleic acid probe that hybridizes with a nucleic acid of a pathogenic microorganism associated with the disease and a second nucleic acid probe that hybridizes with a target nucleic acid of said individual that is associated with susceptibility to said disease; and
b) detecting said nucleic acid of said pathogenic microorganism that binds to said first nucleic acid probe and said nucleic acid of said individual that binds to said second nucleic acid probe, wherein the presence of both said nucleic acid of said pathogenic microorganism that binds to said first nucleic acid probe and said nucleic acid of said individual that binds to said second nucleic acid probe is correlated with susceptibility of said individual to said disease.

30. The method of claim 29, wherein the probes are immobilized on a substrate.

31. The method of claim 29, further comprising the step of amplifying said nucleic acid from said individual prior to reacting in a).

32. A method of preparing a probe-immobilized substrate comprising a first probe that detects the presence of a specific nucleic acid of a pathogenic microorganism associated with a disease and a second probe that detects the presence of a specific nucleic acid of an individual that is associated with responsiveness to a treatment for the disease, comprising the step of immobilizing the first probe and the second probe to a substrate.

33. The method of claim 32, wherein said substrate is selected from the group consisting of a base plate, a porous material, a micro-titer plate, beads, spherical material, a granular material, a magnetic material or magnetic beads.
